# EUROPEAN PATENT SPECIFICATION

(11) **EP 3 867 397 B1**
(45) Date of publication and mention of the grant of the patent: **14.01.2026**
(21) Application number: 19872713.3
(22) Date of filing: 16.10.2019
(51) Int. Cl.: C12Q 1/6806, C12Q 1/6858

(54) **METHODS AND REAGENTS FOR EFFICIENT GENOTYPING OF LARGE NUMBERS OF SAMPLES VIA POOLING**
VERFAHREN UND REAGENZIEN ZUR EFFIZIENTEN GENOTYPISIERUNG VON GROSSEN ZAHLEN VON PROBEN ÜBER POOLING
PROCÉDÉS ET RÉACTIFS POUR LE GÉNOTYPAGE EFFICACE DE GRANDS NOMBRES D'ÉCHANTILLONS PAR REGROUPEMENT

(30) Priority: 16.10.2018 US 201862746543 P
(43) Date of publication of application: 25.08.2021
(73) Proprietor: Twinstrand Biosciences, Inc., Seattle, WA 98121 (US)
(72) Inventor: SALK, Jesse J., Seattle, Washington 98125 (US); DANAHER, Patrick, Seattle, Washington 98115 (US); VALENTINE, Charles Clinton, III, Seattle, Washington 98122 (US)
(74) Representative: HGF
(86) International application number: PCT/US2019/056618
(87) International publication number: WO 2020/081743

(56) References cited:
- WO-A1-2013/142389
- US-A1- 2016 333 416
- US-A1- 2016 333 416
- ANANTHARAMAN RAMANI ET AL: "Validation of pooled genotyping on the Affymetrix 500 k and SNP6.0 genotyping platforms using the polynomial-based probe-specific correction", BMC GENETICS, BIOMED CENTRAL, GB, vol. 10, no. 1, 14 December 2009 (2009-12-14), pages 82, XP021065948, ISSN: 1471-2156
- M. W. SCHMITT ET AL: "Detection of ultra-rare mutations by next-generation sequencing", PROCEEDINGS OF THE NATIONAL ACADEMY OF SCIENCES, vol. 109, no. 36, 1 August 2012 (2012-08-01), pages 14508 - 14513, XP055161683, ISSN: 0027-8424, DOI: 10.1073/pnas.1208715109
- ANANTHARAMAN ET AL.: "Validation of pooled genotyping on the Affymetrix 500 k and SNP6.0 genotyping platforms using the polynomial-based probe-specific correction", BMC GENET., vol. 10, no. 1, 14 December 2009 (2009-12-14), pages 82, XP021065948
- TEUMER A. ET AL.: "Comparison of genotyping using pooled DNA samples (allelotyping) and individual genotyping using the affymetrix genome-wide human SNP array 6.0", BMC GENOMICS, vol. 14, no. 1, 26 July 2013 (2013-07-26), pages 506, XP021156184
- CHI ET AL.: "Combining DNA pooling with selective recombinant genotyping for increased efficiency in fine mapping", THEOR APPL GENET., vol. 120, no. 4, 2010, pages 775 - 83, XP019776233
- WONG ET AL.: "Rare Event Detection Using Error-corrected DNA and RNA Sequencing", J VIS EXP., vol. 138, August 2018 (2018-08-01), pages e57509, XP055702617
- HOGERS ET AL.: "SNPSelect: A scalable and flexible targeted sequence-based genotyping solution", PLOS ONE, vol. 13, no. 10, 12 October 2018 (2018-10-12), pages e0205577, XP055702618

## Description

### BACKGROUND

Next generation DNA sequencing (NGS) makes it possible to sequence trillions of DNA bases in a single sequencer run. Although nucleotide throughput of sequencing has increased enormously in the last decade, cost-efficient technologies for multiplexing hundreds or thousands of samples together to capitalize on NGS' massive capacity have lagged. For some applications where the sequencing needs per sample are large (e.g., whole mammalian genomes or exomes), modest multiplexing capacity is sufficient. For samples where the sequencing needs per sample are small (e.g., panels of a few thousand or tens of thousands of base pairs in size), the cost of filling out a sequencer run becomes high, not by the sequencing itself, but by the cost and effort of preparing such large numbers of samples and individually labeling each with a unique index sequence, and then pooling for multiplexed sequencing. For example, applications involving population sequencing for rare inherited variants within a relatively small targeted gene panel, the hundreds or thousands of parallel library preparations is taxing, expensive and often rate limiting. US2016/333416 A1 describes a method for detecting cancer mutations and aneuploidy in chromosomal segments.

### SUMMARY

The invention is set out in the appended set of claims. The present technology relates generally to methods and associated reagents for efficient genotyping of multiple samples via pooling. In particular, some embodiments of the technology are directed to utilizing Duplex Sequencing for efficient genotyping of large numbers of samples (e.g., nucleic acid samples, patient samples, tissue samples, blood samples, plasma samples, serum samples, swabbing samples, scraping samples, cell culture samples, microbial samples etc.) and associated applications in accordance with the claims. For example, various embodiments of the present technology include performing Duplex Sequencing methods on pooled nucleic acid samples (e.g., patient DNA samples) to simultaneously sequence all, or targeted sections of the genome in a manner that is efficient (e.g., cost efficient, time efficient) and with high accuracy and sensitivity in accordance with the claims. Such embodiments allow for screening for variant alleles (i.e. genetic variants such as SNVs, MNVs, SNPs, MNPs, INDELs, mutations, structural variants, copy number variants, inversions, rearrangements, etc.) from a pool of a modest or large number of original pooled samples, as well as the identification of an individual sample (or samples) having the variant allele. Various aspects of this technology have many applications in both pre-clinical and clinical disease assessment, screening large sample numbers where relatively infrequent variants are being sought, and others in accordance with the claims. Reference to compositions, systems, computer-readable mediums and kits are provided for illustrative purposes only however they may be used in the method in accordance with the claims.

In some embodiments, the present disclosure provides methods for genotyping a plurality of biological samples via pooling that comprises the steps of pooling the plurality of biological samples, or nucleic acid derivatives of biological samples, into a unique combination of sub-pools, wherein each biological sample comprises target double-stranded DNA molecules; and generating an error-corrected sequence read for each of a plurality of the target double-stranded DNA molecules in the sub-pools in accordance with the claims. Generating an error-corrected sequence read comprises the steps of ligating adapter molecules to the plurality of target double-stranded DNA molecules to generate a plurality of adapter-DNA molecules; for each of a plurality of adapter-DNA molecules, generating a set of copies of an original first strand of the adapter-DNA molecule and a set of copies of an original second strand of the adapter-DNA molecule; sequencing one or more copies of the original first and second strands to provide a first strand sequence and a second strand sequence; and comparing the first strand sequence and the second strand sequence to identify one or more correspondences between the first and second strand sequences. In one embodiment, the method further comprises identifying a donor source of nucleic acid present in the mixture of nucleic acid by deconvolving the error-corrected sequence reads into individual genotypes in accordance with the claims. For example, the method can include identifying a presence of one or more variant alleles from the error-corrected sequence reads; and determining the original biological sample containing the variant allele(s) by identifying the unique combination of sub-pools containing the variant allele(s).

In another embodiment, the present technology provides a method for screening biological sources for a genetic variant that includes aliquoting a plurality of biological samples derived from the biological sources into a unique combination of sub-pools, wherein each biological sample comprises target double-stranded DNA molecules, and wherein each biological sample is aliquoted into more than one sub-pool in accordance with the claims. The method further includes generating an error-corrected sequence read for each of a plurality of the target double-stranded DNA molecules in the sub-pools; identifying a presence of one or more variant allele(s) from the error-corrected sequence reads; and determining the biological source containing the variant allele(s) by identifying the unique combination of sub-pools containing the variant allele(s).

In one embodiment, generating an error-corrected sequence read for each of a plurality of the target double-stranded DNA molecules in the sub-pools can further comprise selectively enriching one or more targeted genomic regions prior to sequencing. In some embodiments, the one or more targeted genomic regions comprise genes known to harbor disease-causing mutations. In one embodiment, a disease-causing mutation is or includes a loss of function mutation, a gain of function mutation, or a dominant negative mutation. In another embodiment, the one or more targeted genomic regions comprise genetic loci known to be associated with a disease or disorder. In one embodiment, the disease or disorder is a rare genetic disorder. In one embodiment, the disease or disorder is a single-gene disorder. In another embodiment, the disease or disorder is a complex disorder involving mutations in two or more genes. In one embodiment, the disease or disorder is associated with an autosomal recessive mutation. In another embodiment, the disease or disorder is associated with an autosomal dominant mutation.

In some embodiments, the one or more targeted genomic regions comprise a cancer driver, a proto-oncogene, a tumor suppressor gene and/or an oncogene. In examples, the cancer driver comprises ABL, ACC, BCR, BLCA, BRCA, CESC, CHOL, COAD, DLBC, DNMT3A, EGFR, ESCA, GBM, HNSC, KICH, KIRC, KIRP, LAML, LGG, LIHC, LUAD, LUSC, MESO, OV, PAAD, PCPG, PI3K, PIK3CA, PRAD, PTEN, RAS, READ, SARC, SKCM, STAD, TGCT, THCA, THYM, TP53, UCEC, UCS, and/or UVM. In another embodiment, the one or more targeted genomic regions comprise a gene associated with a rare autoimmune, metabolic or neurological genetic disorder or disease.

In some embodiments, the rare genetic disorder or disease is or comprises Phenylketonuria (PKU), Cystic fibrosis, Sickle-cell anemia, Albinism, Huntington's disease, Myotonic dystrophy type 1, Hypercholesterolemia, Neurofibromatosis, Polycystic kidney disease 1 and 2, Hemophilia A, Muscular dystrophy (Duchenne type), Hypophosphatemic rickets, Rett's syndrome, Tay-Sachs disease, Wilson disease, and/or Spermatogenic failure.

In another embodiment, the one or more targeted genomic regions comprise a genetic locus associated with rare genetic disorders of obesity. In some embodiments, the rare genetic disorders of obesity are or include Proopiomelanocortin (POMC) Deficiency Obesity, Alström syndrome, Leptin Receptor (LEPR) Deficiency Obesity, Prader-Willi syndrome (PWS), Bardet-Biedl syndrome (BBS), and high-impact Heterozygous Obesity.

In one embodiment, identifying a presence of one or more variant allele(s) from the error-corrected sequence reads comprises comparing the error-corrected to a reference genome DNA sequence. In one embodiment, the reference genome DNA sequence is a human reference genome DNA sequence.

In some embodiments, methods disclosed herein can further comprise determining a frequency of the one or more variants among the plurality of target double-stranded DNA molecules in each sub-pool. In one embodiment, methods can further comprise determining if a biological source donor of the biological sample comprising the variant allele(s) is heterozygous or homozygous for the variant allele.

In some embodiments, the target double-stranded DNA molecules are extracted from a blood draw taken from a human. In another embodiment, the target double-stranded DNA molecules are extracted from tissue samples (e.g., biopsy samples, etc.).

Certain aspects of the present technology in accordance with the claims are directed to methods for genotyping a plurality of biological samples that include the steps of: aliquoting the plurality of biological samples into a plurality of sub-pools, wherein each biological sample comprises target double-stranded DNA fragments, and wherein no two biological samples are aliquoted into the same combination of sub-pools; generating duplex sequencing data from raw sequencing data, wherein the raw sequencing data is generated from the plurality of sub-pooled biological samples comprising the target double-stranded DNA fragments, and wherein the target double-stranded DNA fragments contain one or more genetic variants; and identifying a donor source of the one or more genetic variants present in the sub-pooled biological samples by identifying the unique combination of sub-pools containing the one or more genetic variants.

In some embodiments, the method further comprises (e.g., for each sub-pool): (a) preparing a sequencing library from the aliquoted biological samples, wherein preparing the sequence library comprises ligating asymmetric adapter molecules to the plurality of target double-stranded DNA fragments in the sub-pool to generate a plurality of adapter-DNA molecules; (b) sequencing first and second strands of the adapter-DNA molecules to provide a first strand sequence read and a second strand sequence read for each adapter-DNA molecule; (c) for each adapter-DNA molecule, comparing the first strand sequence read and the second strand sequence read to identify one or more correspondences between the first and second strand sequence reads to provide the error-corrected sequence reads for each of a plurality of the target double-stranded DNA molecules in the sub-pools.

In one embodiment, and prior to sequencing in step (b), the method further comprises combining the adapter-DNA molecules from the sub-pools. In one embodiment, the adapter molecules have an indexing sequence. In one embodiment, each sub-pool is tagged using a unique indexing sequence. In one embodiment, identifying the unique combination of sub-pools comprises identifying the indexing sequence associated with each genetic variant.

In some embodiments, the methods further comprise cross-referencing the indexing sequence associated with each genetic variant to the combination of sub-pools each biological sample is aliquoted to identify the donor source.

In some embodiments, the number of sub-pools is or comprises 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 42, 45, 47, 50, 52, 55, 57, 60, 62, 65, 67, or 70 sub-pools. In other embodiments, the number of sub-pools is or comprises between about 15 and about 40 sub-pools, between about 30 and about 50 sub-pools, between about 35 and about 55 sub-pools, between about 40 and about 60 sub-pools, or over 60 sub-pools.

Also described is a method for identifying a patient having a rare variant allele among a population of patients. The method comprises the steps: (a) separating a biological sample from each patient in the population into a unique combination of sub-pooled samples, wherein each biological sample comprises nucleic acid fragments; (b) attaching indexing barcodes to a plurality of the nucleic acid fragments in each sub-pooled sample to generate a plurality of indexed sub-pooled samples; (c) combining the indexed sub-pooled samples to provide a pooled set of barcoded nucleic acid molecules; (d) sequencing the pooled set of barcoded nucleic acid molecules; (e) providing error-corrected sequence reads for a plurality of barcoded nucleic acid molecules; (f) grouping error-corrected sequence reads into sub-pooled samples based on the indexing barcodes; (g) identifying a presence of the rare variant allele from the error-corrected sequence reads in each sub-pooled sample; and (h) identifying the patient containing the rare variant allele by identifying the unique combination of sub-pools containing the rare variant allele.

In some examples, the method includes, prior to steps (a)-(h): screening a mixture of patient DNA from the population of patients for the presence of a carrier of a rare variant allele in the population of patients, wherein screening comprises: mixing a biological sample from each patient in the population into one or more pooled samples, wherein each the number of pooled samples is less than the number sub-pooled samples; sequencing a plurality of target DNA molecules from the one or more pooled samples to generate raw sequencing data; generating duplex sequencing data from the raw sequencing data; and identifying the presence of the rare variant allele in the one or more pooled samples from the duplex sequencing data, thereby determining if the population of patients comprises a carrier of the rare variant allele. In one example, the number of pooled samples is 1. In another example, the number of pooled samples is greater than 1, and wherein steps (a)-(h) comprise identifying a patient having the rare variant allele among a population of patients represented in pooled samples with an identified presence of the rare variant allele.

Also described is a method for screening patient DNA samples for rare variant allele(s), the method comprising: aliquoting each patient DNA sample into a unique subset of pooled DNA samples, wherein the number of pooled DNA samples is less than the number of patient DNA samples, and wherein the unique subset of pooled DNA samples comprises a unique sample identifier for each particular patient DNA sample; sequencing one or more target DNA molecules from each pooled DNA sample; generating high accuracy consensus sequences for the target DNA molecules; identifying a presence of a rare variant allele from the high accuracy consensus sequences; identifying a unique subset of pooled DNA samples comprising the rare variant allele to determine the unique sample identifier associated with the rare variant allele; and identifying the patient DNA sample containing the rare variant allele by the unique sample identifier. In one example, the patient DNA samples comprise double-stranded DNA molecules extracted from healthy tissue, a tumor, and/or a blood sample from the patient.

Also described are systems, such as computing systems, for efficiently genotyping multiple samples. In one example, a system comprises a computer network for transmitting information relating to sequencing data and genotype data, wherein the information includes one or more of raw sequencing data, duplex sequencing data, sub-pooled sample mixture information, individual sample information, and genotype information; a client computer associated with one or more user computing devices and in communication with the computer network; a database connected to the computer network for storing a plurality of genotype profiles and user results records; a duplex sequencing module in communication with the computer network and configured to receive raw sequencing data and requests from the client computer for generating duplex sequencing data, group sequence reads from families representing an original double-stranded nucleic acid molecule and compare representative sequences from individual strands to each other to generate duplex sequencing data; and a genotype module in communication with the computer network and configured to identify variant alleles, determine a sub-pool identification for each variant allele present, and calculate relative abundance of the variant allele within each sub-pool to generate genotype data.

In one example, the genotype profiles comprise known disease-associated mutations. In another example, the genotype profiles comprise empirically derived patient genotypes at one or more genomic loci.

The present disclosure further describes examples of non-transitory computer-readable storage medium. In one example, a non-transitory computer-readable storage medium comprises instructions that, when executed by one or more processors, performs a method of any one of the methods described herein. In one example, the non-transitory computer-readable storage medium further includes instructions for correlating a combination of sub-pools comprising a variant allele to an original sample mixing pattern to identify an original source of the variant allele among a population of sources.

In one example, the present disclosure provides a computer system for performing any one of the methods described herein for efficiently genotyping multiple samples. In one example, the system comprises at least one computer with a processor, memory, database, and a non-transitory computer readable storage medium comprising instructions for the processor(s), wherein said processor(s) are configured to execute said instructions to perform operations comprising the methods described herein.

In another example, the present disclosure describes a non-transitory computer-readable medium whose contents cause at least one computer to perform a method for providing duplex sequencing data for double-stranded nucleic acid molecules in a plurality of sub-pooled sample mixtures. In one example, the method comprises receiving raw sequence data from a user computing device; creating a sub-pool-specific data set comprising a plurality of raw sequence reads derived from a plurality of nucleic acid molecules in the sub-pooled sample mixture; grouping sequence reads from families representing an original double-stranded nucleic acid molecule, wherein the grouping is based on a shared single molecule identifier sequence; comparing a first strand sequence read and a second strand sequence read from an original double-stranded nucleic acid molecule to identify one or more correspondences between the first and second strand sequences reads; providing duplex sequencing data for the double-stranded nucleic acid molecules in the sub-pooled sample mixture; identifying one or more genetic variants present within individual double-stranded nucleic acid molecules in each sub-pooled sample mixture; and determining an original biological source of the one or more genetic variants present in the sub-pooled sample mixtures by resolving the unique combination of sub-pooled sample mixtures that comprise the one or more genetic variants In one example, the method further comprises identifying nucleotide positions of non-complementarity between the compared first and second sequence reads, wherein the method further comprises, in positions of non-complementarity, identifying and eliminating or discounting process errors.

In one example, the step of determining an original biological source comprises using a look-up table to identify the original biological source with nucleic acid aliquots in each of the unique combination of sub-pooled sample mixtures for a particular genetic variant.

In another example, the present disclosure describes a non-transitory computer-readable medium whose contents cause at least one computer to perform a method for detecting, identifying and quantifying variant alleles present in sub-pooled nucleic acid mixtures to determine donor biological sources of the variant alleles. In one example, the method comprises identifying the combination of sub-pooled nucleic acid mixtures comprising a particular variant allele; summing total counts of the particular variant allele within each sub-pooled nucleic acid mixture; and using a look-up table to identify a donor biological source having nucleic acid aliquots in each of the sub-pooled nucleic acid mixtures comprising the particular variant allele. In one example the method further comprises determining whether the donor biological source is heterozygous or homozygous for the particular variant allele based on the total counts of the particular variant allele within each sub-pooled nucleic acid mixture.

In one example, and wherein if any sub-pooled nucleic acid mixture comprises a particular variant allele from more than one donor biological source, the method further comprises differentiating between the more than one donor biological source with a single nucleotide polymorphism (SNP), wherein the SNP is in genomic proximity to a variant sequence on the particular variant allele, and wherein the SNP is not in perfect disequilibrium with the variant sequence. In another example, the total counts of the particular variant allele within each sub-pooled nucleic acid mixture can inform how many donor biological sources of the particular variant allele are present.

In a further example, the present disclosure describes a non-transitory computer-readable medium whose contents cause at least one computer to perform a method for identifying a patient having a variant allele from among a patient population. In one example, the method comprises identifying a variant allele present within individual DNA molecules in a mixture; identifying a combination of sub-pools comprising the identified variant allele, wherein the combination of sub-pools is a subset of a plurality of sub-pools; and identifying the patient among the population of patients having the variant allele by determining which patient donated DNA molecules to the combination of sub-pools comprising the identified variant allele. In one example, the step of identifying the patient comprises using a look-up table correlating a patient DNA sample with a combination of sub-pools.

### BRIEF DESCRIPTION OF THE DRAWINGS

Many aspects of the present disclosure can be better understood with reference to the following drawings. The components in the drawings are not necessarily to scale. Instead, emphasis is placed on illustrating clearly the principles of the present disclosure.
FIG. **1A** illustrates a nucleic acid adapter molecule for use with some embodiments of the present technology and a double-stranded adapter-nucleic acid complex resulting from ligation of the adapter molecule to a double-stranded nucleic acid fragment in accordance with an embodiment of the present technology and in accordance with the claims.
FIGS. **1B** and **1C** are conceptual illustrations of various Duplex Sequencing method steps in accordance with an embodiment of the present technology and in accordance with the claims.
FIG. **2** is a flow diagram of a method for efficient genotyping of a large number of samples in accordance with an embodiment of the present technology and in accordance with the claims.
FIG. **3** is a plot showing cost and performance metrics for sample pooling schemes in accordance with an embodiment of the present technology and in accordance with the claims.
FIG. **4****,** panels **A-D** show a pooling schematic (panel **A),** a look-up table generated from the pooling scheme (panel **B),** a look-up table generated from a sub-pool indexing scheme (panel **C),** and identification of sub-pools containing a variant allele (panel **D)** that can be used with look-up tables (panels **B** and **C)** to identify a patient carrying the identified genetic variant in accordance with an embodiment of the present technology and in accordance with the claims.
FIG. **5** is a schematic diagram of a network computer system for use with the methods and/or reagents disclosed herein for efficient genotyping of multiple samples in accordance with an example of the present technology.
FIG. **6** is a flow diagram illustrating a routine for providing Duplex Sequencing consensus sequence data in accordance with an embodiment of the present technology in accordance with an embodiment of the present technology and in accordance with the claims.
FIG. **7** is a flow diagram illustrating a routine for detecting, identifying and quantifying variant alleles present in nucleic acid mixtures to identify an original contributing source of the variant allele(s) in accordance with an embodiment of the present technology and in accordance with the claims.

### DETAILED DESCRIPTION

The present technology relates generally to methods and associated reagents for efficient genotyping of large numbers of samples via pooling in accordance with the claims. In particular, some embodiments of the technology are directed to utilizing Duplex Sequencing for efficient genotyping of large numbers of samples (e.g., nucleic acid samples, patient samples, tissue samples, blood samples, plasma samples, serum samples, swabbing samples, scraping samples, cell culture samples, microbial samples etc.) and associated applications in accordance with the claims. For example, various embodiments of the present technology include performing Duplex Sequencing methods on pooled nucleic acid samples (e.g., patient DNA samples) to simultaneously sequence all, or targeted sections of the genome in a manner that is efficient (e.g., cost efficient, time efficient) and with high accuracy and sensitivity in accordance with the claims. Some embodiments presented herein and in accordance with the claims allow for genotyping and/or screening biological sources (e.g., patients) for variant alleles using combinations of a subset of pooled samples as unique sample identifiers such that the identification of an individual sample having the variant allele can be identified. Some examples presented herein allows for efficient genotyping and/or screening of artificial sources (e.g., synthetic oligonucleotides, gene-edited samples, manufactured cell population, manufactured viral samples, etc.). Various aspects of the present technology have many applications in both pre-clinical and clinical disease assessment, screening large sample numbers where relatively uncommon variants are being sought within a larger population of independent samples, providing early intervention therapies to patients, and others in accordance with the claims. Various aspects of the present technology also have many applications in biological research, biological manufacturing, high-throughput screening of individual organisms (i.e. humans, animals, plants, fungi) microbial, viral, bacterial, protozoal populations (such as colonies, etc.), and in other fields of genetics in accordance with the claims.

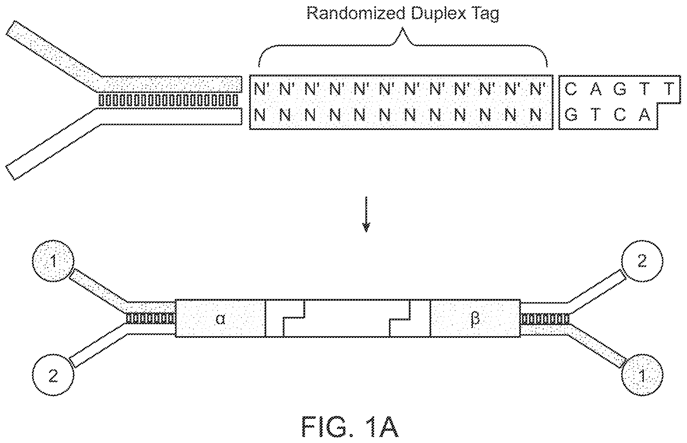

Specific details of several embodiments of the technology are described below with reference to FIGS. 1A-7 and in accordance with the claims. The embodiments can include, for example, methods for pooling/mixing samples and source identification and associated reagents for use in such methods in accordance with the claims. Some embodiments of the technology are directed to utilizing Duplex Sequencing for screening for a presence of variant alleles and variant allele frequency (VAF) in pooled samples in accordance with the claims. Other embodiments of the technology are directed to utilizing Duplex Sequencing for efficient genotyping of large numbers (generally, but not necessarily, 10 or more) of samples (e.g., nucleic acid samples, patient samples, tissue samples, blood samples, etc.) while being able to maintain original source identification with high levels of confidence without necessitating individual labeling of each sample in accordance with the claims. Additional embodiments of the technology are directed to identifying one or more contributing samples among a large number of pooled samples having variant alleles (e.g., identifying the source of the genetic material containing the variant allele(s in accordance with the claims). In further embodiments, the technology is directed to Duplex Sequencing for economically and reliably identifying carriers of rare disease-associated or other trait-associated alleles amongst a population, such as a large population or, in some embodiments, a particular patient population in accordance with the claims.

Although many of the embodiments are described herein with respect to Duplex Sequencing, other sequencing modalities capable of generating error-corrected sequencing reads in addition to those described herein are within the scope of the present technology in accordance with the claims. For example, many embodiments of single-strand consensus sequencing and/or combinations of single and duplex consensus sequencing are contemplated for use in accordance with the claims. A non-exhaustive list of such technologies is described or referenced in Salk et. al. (2018), Enhancing the accuracy of next-generation sequencing for detecting rare and subclonal mutations, Nat Rev Genet, 19, 269-285 (PMID 29576615) and Salk, J.J. and Kennedy, S.R., 2019, Next-Generation Genotoxicology: Using Modern Sequencing Technologies to Assess Somatic Mutagenesis and Cancer Risk, Environ Mol Mutagen., (PMID 31595553). Additionally, other embodiments of the present technology can have different configurations, components, or procedures than those described herein in accordance with the claims. A person of ordinary skill in the art, therefore, will accordingly understand that the technology can have other embodiments with additional elements and that the technology can have other embodiments without several of the features shown and described below with reference to FIGS. 1A-7 in accordance with the claims.

### I. Certain Definitions

In order for the present disclosure to be more readily understood, certain terms are first defined below. Additional definitions for the following terms and other terms are set forth throughout the specification. All definitions are to be understood in accordance with the claims.

In this application, unless otherwise clear from context, the term "a" may be understood to mean "at least one." As used in this application, the term "or" may be understood to mean "and/or." In this application, the terms "comprising" and "including" may be understood to encompass itemized components or steps whether presented by themselves or together with one or more additional components or steps. Where ranges are provided herein, the endpoints are included. As used in this application, the term "comprise" and variations of the term, such as "comprising" and "comprises," are not intended to exclude other additives, components, integers or steps.

***About:*** The term "about", when used herein in reference to a value, refers to a value that is similar, in context to the referenced value. In general, those skilled in the art, familiar with the context, will appreciate the relevant degree of variance encompassed by "about" in that context. For example, in some embodiments, the term "about" may encompass a range of values that within 25%, 20%, 19%, 18%, 17%, 16%, 15%, 14%, 13%, 12%, 11%, 10%, 9%, 8%, 7%, 6%, 5%, 4%, 3%, 2%, 1%, or less of the referred value. For variances of single digit integer values where a single numerical value step in either the positive or negative direction would exceed 25% of the value, "about" is generally accepted by those skilled in the art to include, at least 1, 2, 3, 4, or 5 integer values in either the positive or negative direction, which may or may not cross zero depending on the circumstances. A non-limiting example of this is the supposition that 3 cents can be considered about 5 cents in some situations that would be apparent to one skilled in that art.

***Allele:*** As used herein, the term "allele" refers to one of two or more existing genetic variants of a specific genomic locus.

***Analog:*** As used herein, the term "analog" refers to a substance that shares one or more particular structural features, elements, components, or moieties with a reference substance. Typically, an "analog" shows significant structural similarity with the reference substance, for example sharing a core or consensus structure, but also differs in certain discrete ways. In some embodiments, an analog is a substance that can be generated from the reference substance, e.g., by chemical manipulation of the reference substance. In some embodiments, an analog is a substance that can be generated through performance of a synthetic process substantially similar to (e.g., sharing a plurality of steps with) one that generates the reference substance. In some embodiments, an analog is or can be generated through performance of a synthetic process different from that used to generate the reference substance.

***Animal:*** as used herein refers to any member of the animal kingdom. In some embodiments, "animal" refers to humans, of either sex and at any stage of development. In some embodiments, "animal" refers to non-human animals, at any stage of development. In certain embodiments, the non-human animal is a mammal (e.g., a rodent, a mouse, a rat, a rabbit, a monkey, a dog, a cat, a sheep, cattle, a primate, and/or a pig). In some embodiments, animals include, but are not limited to, mammals, birds, reptiles, amphibians, fish, insects, and/or worms. In some embodiments, an animal may be a transgenic animal, genetically engineered animal, and/or a clone.

***Biological Sample:*** As used herein, the term "biological sample" or "sample" typically refers to a sample obtained or derived from one or more biological sources (e.g., a tissue or organism or cell culture) of interest, as described herein. In some embodiments, a source of interest comprises an organism, such as an animal or human. In other embodiments, a source of interest comprises a microorganism, such as a bacterium, virus, protozoan, or fungus. In further embodiments, a source of interest may be a synthetic tissue, organism, cell culture, nucleic acid or other material. In yet further embodiments, a source of interest may be a plant-based organism. In yet another embodiment, a sample may be an environmental sample such as, for example, a water sample, soil sample, archeological sample, or other sample collected from a non-living source. In other embodiments, a sample may be a multi-organism sample (e.g., a mixed organism sample). In still further embodiments, a sample may comprise a cell mixture or a tissue mixture. In other embodiments, a sample may be derived from a multichimeric organism or tissue, transplant tissue, or multichimeric cell cultures. In further embodiments, the sample may include fetal DNA. In yet other embodiments, a sample may be collected from a crime scene or other law enforcement investigation inquiry (e.g., in forensic cases such as for identifying perpetrators, victims or missing persons, etc.). In other embodiments, a sample may be collected from a war or terrorism investigation inquiry or historical study (e.g., for identifying victims or missing persons), etc. In other embodiments, a sample may be collected from an archeological study. In some embodiments, a biological sample is or comprises biological tissue or fluid. In some embodiments, a biological sample may be isolated DNA or other nucleic acids or may comprise bone marrow; blood; blood cells; stem cells, ascites; tissue samples, biopsy samples or or fine needle aspiration samples; cell-containing body fluids; free floating nucleic acids; protein-bound nucleic acids, riboprotein-bound nucleic acids; sputum; saliva; urine; cerebrospinal fluid, peritoneal fluid; pleural fluid; feces; lymph; gynecological fluids; skin swabs; vaginal swabs; pap smear, oral swabs; nasal swabs; washings or lavages such as a ductal lavages or broncheoalveolar lavages; vaginal fluid, aspirates; scrapings; bone marrow specimens; tissue biopsy specimens; fetal tissue or fluids; surgical specimens; feces, other body fluids, secretions, and/or excretions; and/or cells therefrom, *etc.* In some embodiments, a biological sample is or comprises cells obtained from an individual. In some embodiments, obtained cells are or include cells from an individual from whom the sample is obtained. In some embodiments cell-derivatives such as organelles or vesicles or exosomes. In a particular embodiment, a biological sample is a liquid biopsy obtained from a subject. In some embodiments, a sample is a "primary sample" obtained directly from a source of interest by any appropriate means. For example, in some embodiments, a primary biological sample is obtained by methods selected from the group consisting of biopsy (*e.g.,* fine needle aspiration or tissue biopsy), surgery, collection of body fluid (*e.g.,* blood (or plasma or serum separated therefrom), lymph, feces *etc*.), *etc.* In some embodiments, as will be clear from context, the term "sample" refers to a preparation that is obtained by processing (e.g., by removing one or more components of and/or by adding one or more agents to) a primary sample. For example, filtering using a semi-permeable membrane. Such a "processed sample" may comprise, for example nucleic acids or proteins extracted from a sample or obtained by subjecting a primary sample to techniques such as amplification or reverse transcription of mRNA, isolation and/or purification of certain components, *etc.*

***Cancer disease:*** In an embodiment, a disease or disorder is a "cancer disease" which is familiar to those experience in the art as being generally characterized by dysregulated growth of abnormal cells, which may metastasize. Cancer diseases detectable using one or more aspects of the present technology comprise, by way of non-limiting examples, prostate cancer (i.e. adenocarcinoma, small cell), ovarian cancer (e.g., ovarian adenocarcinoma, serous carcinoma or embryonal carcinoma, yolk sac tumor, teratoma), liver cancer (e.g., HCC or hepatoma, angiosarcoma), plasma cell tumors (e.g., multiple myeloma, plasmacytic leukemia, plasmacytoma, amyloidosis, Waldenstrom's macroglobulinemia), colorectal cancer (e.g., colonic adenocarcinoma, colonic mucinous adenocarcinoma, carcinoid, lymphoma and rectal adenocarcinoma, rectal squamous carcinoma), leukemia (e.g., acute myeloid leukemia, acute lymphocytic leukemia, chronic myeloid leukemia, chronic lymphocytic leukemia, acute myeloblastic leukemia, acute promyelocytic leukemia, acute myelomonocytic leukemia, acute monocytic leukemia, acute erythroleukemia, and chronic leukemia, T-cell leukemia, Sezary syndrome, systemic mastocytosis, hairy cell leukemia, chronic myeloid leukemia blast crisis), myelodysplastic syndrome, lymphoma (e.g., diffuse large B-cell lymphoma, cutaneous T-cell lymphoma, peripheral T-cell lymphoma, Hodgkin's lymphoma, non-Hodgkin's lymphoma, follicular lymphoma, mantle cell lymphoma, MALT lymphoma, marginal cell lymphoma, Richter's transformation, double hit lymphoma, transplant associated lymphoma, CNS lymphoma, extranodal lymphoma, HIV-associated lymphoma, hairy cell leukemia, variant hairy cell leukemia, endemic lymphoma, Burkitt's lymphoma, transplant-associated lymphoproliferative neoplasms, and lymphocytic lymphoma etc.), cervical cancer (squamous cervical carcinoma, clear cell carcinoma, HPV associated carcinoma, cervical sarcoma etc.) esophageal cancer (esophageal squamous cell carcinoma, adenocarcinoma, certain grades of Barretts esophagus, esophageal adenocarcinoma), melanoma (dermal melanoma, uveal melanoma, acral melanoma, amelanotic melanoma etc.), CNS tumors (e.g., oligodendroglioma, astrocytoma, glioblastoma multiforme, meningioma, schwannoma, craniopharyngioma etc.), pancreatic cancer (e.g., adenocarcinoma, adenosquamous carcinoma, signet ring cell carcinoma, hepatoid carcinoma, colloid carcinoma, islet cell carcinoma, pancreatic neuroendocrine carcinoma etc.), gastrointestinal stromal tumor, sarcoma (e.g., fibrosarcoma, myxosarcoma, liposarcoma, chondrosarcoma, osteogenic sarcoma, angiosarcoma, endothelioma sarcoma, lymphangiosarcoma, lymphangioendothelioma sarcoma, leiomyosarcoma, Ewing's sarcoma, and rhabdomyosarcoma, spindle cell tumor etc.), breast cancer (e.g., inflammatory carcinoma, lobar carcinoma, ductal carcinoma etc.), ER-positive cancer, HER-2 positive cancer, bladder cancer (squamous bladder cancer, small cell bladder cancer, urothelial cancer etc.), head and neck cancer (e.g., squamous cell carcinoma of the head and neck, HPV-associated squamous cell carcinoma, nasopharyngeal carcinoma etc.), lung cancer (e.g., non-small cell lung carcinoma, large cell carcinoma, bronchogenic carcinoma, squamous cell cancer, small cell lung cancer etc.), metastatic cancer, oral cavity cancer, uterine cancer (leiomyosarcoma, leiomyoma etc.), testicular cancer (e.g., seminoma, non-seminoma, and embryonal carcinoma yolk sack tumor etc), skin cancer (e.g., squamous cell carcinoma, and basal cell carcinoma, merkel cell carcinoma, melanoma, cutaneous t-cell lymphoma etc.), thyroid cancer (e.g., papillary carcinoma, medullary carcinoma, anaplastic thyroid cancer etc.), stomach cancer, intra-epithelial cancer, bone cancer, biliary tract cancer, eye cancer, larynx cancer, kidney cancer (e.g., renal cell carcinoma, Wilms tumor etc.), gastric cancer, blastoma (e.g., nephroblastoma, medulloblastoma, hemangioblastoma, neuroblastoma, retinoblastoma etc.), myeloproliferative neoplasms (polycythemia vera, essential thrombocytosis, myelofibrosis, etc.), chordoma, synovioma, mesothelioma, adenocarcinoma, sweat gland carcinoma, sebaceous gland carcinoma, cystadenocarcinoma, bile duct carcinoma, choriocarcinoma, epithelial carcinoma, ependymoma, pinealoma, acoustic neuroma, schwannoma, meningioma, pituitary adenoma, nerve sheath tumor, cancer of the small intestine, pheochromocytoma, small cell lung cancer, peritoneal mesothelioma, hyperparathyroid adenoma, adrenal cancer, cancer of unknown primary, cancer of the endocrine system, cancer of the penis, cancer of the urethra, cutaneous or intraocular melanoma, a gynecologic tumor, solid tumors of childhood, or neoplasms of the central nervous system, primary mediastinal germ cell tumor, clonal hematopoiesis of indeterminate potential, smoldering myeloma, monoclonal gammaglobulinopathy of unknown significant, monoclonal B-cell lymphocytosis, low grade cancers, clonal field defects, preneoplastic neoplasms, ureteral cancer, autoimmune-associated cancers (i.e. ulcerative colitis, primary sclerosing cholangitis, celiac disease), cancers associated with an inherited predisposition (i.e. those carrying genetic defects in such as *BRCA1, BRCA2, TP53, PTEN, ATM,* etc.) and various genetic syndromes such as MEN1, MEN2 trisomy 21 etc.) and those occurring when exposed to chemicals in utero (i.e. clear cell cancer in female offspring of women exposed to Diethylstilbestrol [DES]), among many others.

***Carrier:*** As used herein, "carrier" refers to a subject that has a variant allele of interest (e.g., an allele having a genetic variant, mutation, polymorphism etc.). A human subject or other diploid organism can be identified as a homozygous carrier when both alleles (i.e. maternal derived and paternal derived) in somatic cells contain the genetic variant, or can be identified as a heterozygous carrier when only one allele comprises the genetic variant (e.g., the two alleles do not have the same sequence). A human subject or other diploid organism can be identified as a compound heterozygote where the two alleles each carry a genetic variant that differs from that of a reference sequence but also differ from each other. In some embodiments, depending on the functional consequence of a particular variant (i.e. recessive), the term "carrier" may more commonly used to refer to only the heterozygous state, and homozygotes are referred to as "affected" or as "having disease" or "having trait", in contrast to only one allele. In other embodiments (such as a dominant genetic disease) a heterozygous subject may be more commonly referred to as "affected" or "having disease" or "having trait", in contrast to being described as a carrier. One skilled in the art will recognize the common or accepted scientific or clinical use of the term may vary from situation and are not limited exclusively to the definitions above. In the cases of non-diploid organisms, aneuploidy, loss-of heterozygosity and epigenetic silencing or activation of one allele (i.e. lyonization), and other unique biological situations will be recognized by one skilled in the art as a potential basis for different usages of the term.

***Determine:*** Many methodologies described herein include a step of "determining". Those of ordinary skill in the art, reading the present specification, will appreciate that such "determining" can utilize or be accomplished through use of any of a variety of techniques available to those skilled in the art, including for example specific techniques explicitly referred to herein. In some embodiments, determining involves manipulation of a physical sample. In some embodiments, determining involves consideration and/or manipulation of data or information, for example utilizing a computer or other processing unit adapted to perform a relevant analysis. In some embodiments, determining involves receiving relevant information and/or materials from a source. In some embodiments, determining involves comparing one or more features of a sample or entity to a comparable reference database or that of a reference sample or reference region elsewhere in the genome under examination.

***Duplex Sequencing (DS):*** As used herein, "Duplex Sequencing (DS)" is, in its broadest sense, refers to a tag-based error-correction method that achieves exceptional accuracy by comparing the sequence from both strands of individual DNA molecules double-stranded nucleic acid molecules, most commonly,.

***Expression:*** As used herein, "expression" of a nucleic acid sequence refers to one or more of the following events: (1) production of an RNA template from a DNA sequence (e.g., by transcription); (2) processing of an RNA transcript (e.g., by splicing, editing, 5' cap formation, and/or 3' end formation); (3) translation of an RNA into a polypeptide or protein; and/or (4) post-translational modification of a polypeptide or protein.

***Gene:*** As used herein, the term "gene" refers to a DNA sequence in a chromosome that codes for a product (e.g., an RNA product and/or a polypeptide product). In some embodiments, a gene includes coding sequence (i.e., sequence that encodes a particular product); in some embodiments, a gene includes non-coding sequence. In some particular embodiments, a gene may include both coding (e.g., exonic) and non-coding (e.g., intronic) sequences. In some embodiments, a gene may include one or more regulatory elements that, for example, may control or impact one or more aspects of gene expression (e.g., cell-type-specific expression, inducible expression, etc.).

***Homology:*** As used herein, the term "homology" refers to the overall relatedness between polymeric molecules, e.g., between nucleic acid molecules (e.g., DNA molecules and/or RNA molecules). In some embodiments, polymeric molecules are considered to be "homologous" to one another if their sequences are at least 80%, 85%, 90%, 95%, or 99% identical. As will be understood by those skilled in the art, a variety of algorithms are available that permit comparison of sequences in order to determine their degree of homology, including by permitting gaps of designated length in one sequence relative to another when considering which residues "correspond" to one another in different sequences. Calculation of the percent homology between two nucleic acid sequences, for example, can be performed by aligning the two sequences for optimal comparison purposes (e.g., gaps can be introduced in one or both of a first and a second nucleic acid sequences for optimal alignment and non-corresponding sequences can be disregarded for comparison purposes). In certain embodiments, the length of a sequence aligned for comparison purposes is at least 30%, at least 40%, at least 50%, at least 60%, at least 70%, at least 80%, at least 90%, at least 95%, or substantially 100% of the length of the reference sequence. The nucleotides at corresponding nucleotide positions are then compared. When a position in the first sequence is occupied by the same nucleotide as the corresponding position in the second sequence, then the molecules are identical at that position; when a position in the first sequence is occupied by a similar nucleotide as the corresponding position in the second sequence, then the molecules are similar at that position. The percent homology between the two sequences is a function of the number of identical and similar positions shared by the sequences, taking into account the number of gaps, and the length of each gap, which needs to be introduced for optimal alignment of the two sequences. Algorithms and computer programs useful in determining the percent homology between two nucleotide sequences are well known in the art. In some embodiments, "homology" may refer to the degree of relatedness of the polypeptide produced between two different coding sequences or the degree of structural relatedness of the protein or ribozyme or aptamer that is coded for by two or more nucleic acid sequences, as will be understood by one experienced in the art.

***Mutation:*** As used herein, the term "mutation" refers to alterations to nucleic acid sequence or structure. Mutations to a polynucleotide sequence can include point mutations (e.g., single base mutations, SNPs, SNVs), multinucleotide mutations (MNPs, MNVs), nucleotide deletions, sequence rearrangements, nucleotide insertions, and duplications of the DNA sequence in the sample, inversions, among complex multinucelotide changes. Mutations can occur on both strands of a duplex DNA molecule as complementary base changes (i.e. true mutations), or as a mutation on one strand but not the other strand (i.e. heteroduplex), that has the potential to be either repaired, destroyed or be mis-repaired/converted into a true double stranded mutation. Mutations may represent alterations relative to a control sample from the same or a related source and/or individual or another individual or sample. Mutations may represent alterations relative to a reference sequence.

***Non-cancerous disease:*** In another embodiment, a disease or disorder is a non-cancerous disease that is caused by, or contributed to by, a genomic mutation or damage. By way of non-limiting examples, such non-cancerous types of diseases or disorders that are detectable using one or more aspects of the present technology comprise certain forms of inherited metabolic disorders, cystic fibrosis, hemoglobinopathies, muscular dystrophies, thalasemias, porpheryas, hypertrophic cardiomyopathies, and inherited propensity towards diabetes, autoimmune disease or disorders, infertility, neurodegeneration, cardiovascular disease, Alzheimer's/dementia, obesity, heart disease, high blood pressure, arthritis, mental illness, other neurological disorders, among many other multifactorial inherited disorders (e.g., a predisposition to be more easily triggered by environmental factors). As will be understood by on experienced in the art, some non-cancerous diseases have no known associated genetic component.

***Nucleic acid:*** As used herein, in its broadest sense, refers to any compound and/or substance that is or can be incorporated into an oligonucleotide chain. In some embodiments, a nucleic acid is a compound and/or substance that is or can be incorporated into an oligonucleotide chain via a phosphodiester linkage. As will be clear from context, in some embodiments, *"nucleic acid"* refers to an individual nucleic acid residue (e.g., a nucleotide and/or nucleoside); in some embodiments, *"nucleic acid"* refers to an oligonucleotide chain comprising individual nucleic acid residues. In some embodiments, a *"nucleic acid"* is or comprises RNA; in some embodiments, a *"nucleic acid"* is or comprises DNA. In some embodiments, a nucleic acid is, comprises, or consists of one or more natural nucleic acid residues. In some embodiments, a nucleic acid is, comprises, or consists of one or more nucleic acid analogs. In some embodiments, a nucleic acid analog differs from a nucleic acid in that it does not utilize a phosphodiester backbone. For example, in some embodiments, a nucleic acid is, comprises, or consists of one or more *"peptide nucleic acids",* which are known in the art and have peptide bonds instead of phosphodiester bonds in the backbone, are considered within the scope of the present technology. Alternatively, or additionally, in some embodiments, a nucleic acid has one or more phosphorothioate and/or 5'-N-phosphoramidite linkages rather than phosphodiester bonds. In some embodiments, a nucleic acid is, comprises, or consists of one or more natural nucleosides (e.g., adenosine, thymidine, guanosine, cytidine, uridine, deoxyadenosine, deoxythymidine, deoxy guanosine, and deoxycytidine). In some embodiments, a nucleic acid is, comprises, or consists of one or more nucleoside analogs (e.g., 2-aminoadenosine, 2-thiothymidine, inosine, pyrrolo-pyrimidine, 3 -methyl adenosine, 5-methylcytidine, C-5 propynyl-cytidine, C-5 propynyl-uridine, 2-aminoadenosine, C5-bromouridine, C5-fluorouridine, C5-iodouridine, C5-propynyl-uridine, C5 -propynyl-cytidine, C5-methylcytidine, 2-aminoadenosine, 7-deazaadenosine, 7-deazaguanosine, 8-oxoadenosine, 8-oxoguanosine, 0(6)-methylguanine, 2-thiocytidine, methylated bases, intercalated bases, and combinations thereof). In some embodiments, a nucleic acid comprises one or more modified sugars (e.g., 2'-fluororibose, ribose, 2'-deoxyribose, arabinose, and hexose) as compared with those in natural nucleic acids. In some embodiments, a nucleic acid has a nucleotide sequence that encodes a functional gene product such as an RNA or protein. In some embodiments, a nucleic acid includes one or more introns. In some embodiments, nucleic acids are prepared by one or more of isolation from a natural source, enzymatic synthesis by polymerization based on a complementary template (*in vivo* or *in vitro*), reproduction in a recombinant cell or system, and chemical synthesis. In some embodiments, a nucleic acid is at least 2, 3, 4, 5, 6, 7, 8, 9, 10, 15, 20, 25, 30, 35, 40, 45, 50, 55, 60, 65, 70, 75, 80, 85, 90, 95, 100, 1 10, 120, 130, 140, 150, 160, 170, 180, 190, 200, 225, 250, 275, 300, 325, 350, 375, 400, 425, 450, 475, 500, 600, 700, 800, 900, 1000, 1500, 2000, 2500, 3000, 3500, 4000, 4500, 5000 or more residues long. In some embodiments, a nucleic acid is partly or wholly single stranded; in some embodiments, a nucleic acid is partly or wholly double-stranded. In some embodiments a nucleic acid may be branched of have secondary structures. In some embodiments a nucleic acid has a nucleotide sequence comprising at least one element that encodes, or is the complement of a sequence that encodes, a polypeptide. In some embodiments, a nucleic acid has enzymatic activity. In some embodiments the nucleic acid serves a mechanical function, for example in a ribonucleoprotein complex or a transfer RNA.

***Polynucleotide damage:*** As used herein, the term "polynucleotide damage" or "nucleic acid damage" refers to damage to a subject's deoxyribonucleic acid (DNA) sequence ("DNA damage") or ribonucleic acid (RNA) sequence ("RNA damage") that is directly or indirectly (e.g. a metabolite, or induction of a process that is damaging or mutagenic) caused by an agent or process. Damaged nucleic acid may lead to the onset of a disease or disorder in a subject. Polynucleotide damage may further comprise chemical and/or physical modification of the DNA in a cell. In some embodiments, the damage is or comprises, by way of non-limiting examples, at least one of oxidation, alkylation, deamination, methylation, hydrolysis, hydroxylation, nicking, intra-strand crosslinks, inter-strand cross links, blunt end strand breakage, staggered end double strand breakage, phosphorylation, dephosphorylation, sumoylation, glycosylation, deglycosylation, putrescinylation, carboxylation, halogenation, formylation, single-stranded gaps, damage from heat, damage from desiccation, damage from UV exposure, damage from gamma radiation damage from X-radiation, damage from ionizing radiation, damage from non-ionizing radiation, damage from heavy particle radiation, damage from nuclear decay, damage from beta-radiation, damage from alpha radiation, damage from neutron radiation, damage from proton radiation, damage from antimatter, damage from cosmic radiation, damage from high pH, damage from low pH, damage from reactive oxidative species, damage from free radicals, damage from peroxide, damage from hypochlorite, damage from tissue fixation such formalin or formaldehyde, damage from reactive iron, damage from low ionic conditions, damage from high ionic conditions, damage from unbuffered conditions, damage from nucleases, damage from environmental exposure, damage from fire, damage from mechanical stress, damage from enzymatic degradation, damage from microorganisms, damage from preparative mechanical shearing, damage from preparative enzymatic fragmentation, damage having naturally occurred *in vivo,* damage having occurred during nucleic acid extraction, damage having occurred during sequencing library preparation, damage having been introduced by a polymerase, damage having been introduced during nucleic acid repair, damage having occurred during nucleic acid end-tailing, damage having occurred during nucleic acid ligation, damage having occurred during sequencing, damage having occurred from mechanical handling of DNA, damage having occurred during passage through a nanopore, damage having occurred as part of aging in an organism, damage having occurred as a result if chemical exposure of an individual, damage having occurred by a mutagen, damage having occurred by a carcinogen, damage having occurred by a clastogen, damage having occurred from *in vivo* inflammation damage due to oxygen exposure, damage due to one or more strand breaks, and any combination thereof.

***Reference:*** As used herein, the term "reference" describes a standard or control relative to which a comparison is performed. For example, in some embodiments, an agent, animal, individual, population, sample, sequence or value of interest is compared with a reference or control agent, animal, individual, population, sample, sequence or value or representation thereof in a physical or computer database that may be present at a location or accessed remotely via electronic means. In one embodiment, the reference is a reference genome or a reference genome assembly. In some embodiments, a reference or control is tested and/or determined substantially simultaneously with the testing or determination of interest. In some embodiments, a reference or control is a historical reference or control, optionally embodied in a tangible medium. Typically, as would be understood by those skilled in the art, a reference or control is determined or characterized under comparable conditions or circumstances to those under assessment. Those skilled in the art will appreciate when sufficient similarities are present to justify reliance on and/or comparison to a particular possible reference or control. A "reference sample" refers to a sample from a subject that is distinct from the test subject and isolated in the same way as the sample to which it is compared. The subject of the reference sample may be genetically identical to the test subject or may be different.

***Single Molecule Identifier (SMI):*** As used herein, the term "single molecule identifier" or "SMI", (which may be referred to as a "tag" a "barcode", a "molecular bar code", a "Unique Molecular Identifier", or "UMI", among other names) refers to any material (e.g., a nucleotide sequence, a nucleic acid molecule feature) that is capable of substantially distinguishing an individual molecule among a larger heterogeneous population of molecules. In some embodiments, a SMI can be or comprise an exogenously applied SMI. In some embodiments, an exogenously applied SMI may be or comprise a degenerate or semi-degenerate sequence. In some embodiments substantially degenerate SMIs may be known as Random Unique Molecular Identifiers (R-UMIs). In some embodiments an SMI may comprise a code (for example a nucleic acid sequence) from within a pool of known codes. In some embodiments pre-defined SMI codes are known as Defined Unique Molecular Identifiers (D-UMIs). In some embodiments, a SMI can be or comprise an endogenous SMI. In some embodiments, an endogenous SMI may be or comprise information related to specific shear-points of a target sequence, features relating to the terminal ends of individual molecules comprising a target sequence, or a specific sequence at or adjacent to or within a known distance from an end of individual molecules. In some embodiments an SMI may relate to a sequence variation in a nucleic acid molecule cause by random or semi-random damage, chemical modification, enzymatic modification or other modification to the nucleic acid molecule. In some embodiments the modification may be deamination of methylcytosine. In some embodiments the modification may entail sites of nucleic acid nicks. In some embodiments, an SMI may comprise both exogenous and endogenous elements. In some embodiments an SMI may comprise physically adjacent SMI elements. In some embodiments SMI elements may be spatially distinct in a molecule. In some embodiments an SMI may be a non-nucleic acid. In some embodiments an SMI may comprise two or more different types of SMI information. Various embodiments of SMIs are further disclosed in International Patent Publication No. WO2017/100441.

***Strand Defining Element (SDE):*** As used herein, the term "Strand Defining Element" or "SDE", refers to any material which allows for the identification of a specific strand of a double-stranded nucleic acid material and thus differentiation from the other/complementary strand (e.g., any material that renders the amplification products of each of the two single stranded nucleic acids resulting from a target double-stranded nucleic acid substantially distinguishable from each other after sequencing or other nucleic acid interrogation). In some embodiments, a SDE may be or comprise one or more segments of substantially non-complementary sequence within an adapter sequence. In particular embodiments, a segment of substantially non-complementary sequence within an adapter sequence can be provided by an adapter molecule comprising a Y-shape or a "loop" shape. In other embodiments, a segment of substantially non-complementary sequence within an adapter sequence may form an unpaired "bubble" in the middle of adjacent complementary sequences within an adapter sequence. In other embodiments an SDE may encompass a nucleic acid modification. In some embodiments an SDE may comprise physical separation of paired strands into physically separated reaction compartments. In some embodiments an SDE may comprise a chemical modification. In some embodiments an SDE may comprise a modified nucleic acid. In some embodiments an SDE may relate to a sequence variation in a nucleic acid molecule caused by random or semi-random damage, chemical modification, enzymatic modification or other modification to the nucleic acid molecule. In some embodiments the modification may be deamination of methylcytosine. In some embodiments the modification may entail sites of nucleic acid nicks. Various embodiments of SDEs are further disclosed in International Patent Publication No. WO2017/100441.

***Subject:*** As used herein, the term "subject" refers an organism, typically a mammal, such as a human (in some embodiments including prenatal human forms), a non-human animal (e.g., mammals and non-mammals including, but not limited to, non-human primates, mice, rats, hamsters, otters, wildebeests, horses, sheep, dogs, cows, pigs, chickens, amphibians, reptiles, sea-life, other model organisms such as worms, flies, zebrafish etc.), and transgenic animals (e.g., transgenic rodents), etc. In some embodiments, a subject is suffering from a relevant disease, disorder or condition. In some embodiments, a subject is susceptible to a disease, disorder, or condition. In some embodiments, a subject displays one or more symptoms or characteristics of a disease, disorder or condition. In some embodiments, a subject does not display any symptom or characteristic of a disease, disorder, or condition. In some embodiments, a subject has one or more features characteristic of susceptibility to or risk of a disease, disorder, or condition. In some embodiments, a subject is an individual to whom diagnosis and/or therapy is and/or has been administered. In still other embodiments, a subject refers to any living biological sources or other nucleic acid material, for example, organisms, cells, and/or tissues, such as for *in vivo* studies, e.g.: fungi, protozoans, bacteria, archaebacteria, viruses, isolated cells in culture, cells that have been intentionally (e.g., stem cell transplant, organ transplant) or unintentionally (i.e. fetal or maternal microchimerism) or isolated nucleic acids or organelles (i.e. mitochondria, chloroplasts, free viral genomes, free plasmids, aptamers, ribozymes or derivatives or precursors of nucleic acids (i.e. oligonucleotides, dinucleotide triphosphates, etc.). In further embodiments, a subject refers to any living, or at one time living biological sources or other nucleic acid materials obtained in a forensic investigation or application.

***Substantially:*** As used herein, the term "substantially" refers to the qualitative condition of exhibiting total or near-total extent or degree of a characteristic or property of interest. One of ordinary skill in the biological arts will understand that biological and chemical phenomena rarely, if ever, go to completion and/or proceed to completeness or achieve or avoid an absolute result. The term "substantially" is therefore used herein to capture the potential lack of completeness inherent in many biological and chemical phenomena.

***Variant:*** As used herein, the term "variant" refers to an entity that shows significant structural identity with a reference entity but differs structurally from the reference entity in the presence or level of one or more chemical moieties as compared with the reference entity. In the context of nucleic acids, a variant nucleic acid may have a characteristic sequence element comprised of a plurality of nucleotide residues having designated positions relative to another nucleic acid in linear or three-dimensional space. For example, a variant polynucleotide (e.g., DNA) may differ from a reference polynucleotide as a result of one or more differences in nucleic acid sequence. In some embodiments, a variant polynucleotide sequence includes an insertion, deletion, substitution or mutation relative to another sequence (e.g., a reference sequence or other polynucleotide (e.g., DNA) sequences in a sample).

***Variant frequency:*** As used herein, the term "variant frequency" refers to the relative frequency of a genetic variant at a particular locus in a population, expressed as a fraction or percentage of the population.

***Variant allele frequency:*** As used herein, the term "variant allele frequency" refers to is the relative frequency of an allele (variant of a gene or other sequence) at a particular locus in a population (e.g., a fraction of all chromosomes in the population that carry that allele).

### II. Selected Embodiments of Duplex Sequencing Methods and Associated Adapters and Reagents

Duplex Sequencing is a method for producing error-corrected DNA sequences from double stranded nucleic acid molecules, and which was originally described in International Patent Publication No. WO 2013/142389 and in U.S. Patent No. 9,752,188, and WO 2017/100441, in Schmitt et. al., PNAS, 2012 [1]; in Kennedy et. al., PLOS Genetics, 2013 [2]; in Kennedy et. al., Nature Protocols, 2014 [3]; and in Schmitt et. al., Nature Methods, 2015 [4]. As illustrated in FIGS. 1A-1C, and in certain aspects of the technology, Duplex Sequencing can be used to independently sequence both strands of individual DNA molecules in such a way that the derivative sequence reads can be recognized as having originated from the same double-stranded nucleic acid parent molecule during massively parallel sequencing (MPS), also commonly known as next generation sequencing (NGS), but also differentiated from each other as distinguishable entities following sequencing. The resulting sequence reads from each strand are then compared for the purpose of obtaining an error-corrected sequence of the original double-stranded nucleic acid molecule known as a Duplex Consensus Sequence (DCS). The process of Duplex Sequencing makes it possible to explicitly confirm that both strands of an original double stranded nucleic acid molecule are represented in the generated sequencing data used to form a DCS.

In certain embodiments in accordance with the claims, methods incorporating DS may include ligation of one or more sequencing adapters to a target double-stranded nucleic acid molecule, comprising a first strand target nucleic acid sequence and a second strand target nucleic sequence, to produce a double-stranded target nucleic acid complex (e.g. FIG. **1A****).**

In various embodiments in accordance with the claims, a resulting target nucleic acid complex can include at least one SMI sequence, which may entail an exogenously applied degenerate or semi-degenerate sequence (e.g., randomized duplex tag shown in FIG. **1A****,** sequences identified as α and β in FIG. **1A****),** endogenous information related to the specific shear-points of the target double-stranded nucleic acid molecule, or a combination thereof. The SMI can render the target-nucleic acid molecule substantially distinguishable from the plurality of other molecules in a population being sequenced either alone or in combination with distinguishing elements of the nucleic acid fragments to which they were ligated. The SMI element's substantially distinguishable feature can be independently carried by each of the single strands that form the double-stranded nucleic acid molecule such that the derivative amplification products of each strand can be recognized as having come from the same original substantially unique double-stranded nucleic acid molecule after sequencing. In other embodiments, the SMI may include additional information and/or may be used in other methods for which such molecule distinguishing functionality is useful, such as those described in the above-referenced publications. In another embodiment, the SMI element may be incorporated after adapter ligation. In some embodiments, the SMI is double stranded in nature. In other embodiments it is single-stranded in nature (e.g., the SMI can be on the single-stranded portion(s) of the adapters). In other embodiments, it is a combination of single-stranded and double-stranded in nature.

In some embodiments in accordance with the claims, each double-stranded target nucleic acid sequence complex can further include an element (e.g., an SDE) that renders the amplification products of the two single-stranded nucleic acids that form the target double-stranded nucleic acid molecule substantially distinguishable from each other after sequencing. In one embodiment, an SDE may comprise asymmetric primer sites comprised within the sequencing adapters, or, in other arrangements, sequence asymmetries may be introduced into the adapter molecules not within the primer sequences, such that at least one position in the nucleotide sequences of the first strand target nucleic acid sequence complex and the second stand of the target nucleic acid sequence complex are different from each other following amplification and sequencing. In other embodiments, the SMI may comprise another biochemical asymmetry between the two strands that differs from the canonical nucleotide sequences A, T, C, G or U, but is converted into at least one canonical nucleotide sequence difference in the two amplified and sequenced molecules. In yet another embodiment, the SDE may be a means of physically separating the two strands before amplification, such that the derivative amplification products from the first strand target nucleic acid sequence and the second strand target nucleic acid sequence are maintained in substantial physical isolation from one another for the purposes of maintaining a distinction between the two. Other such arrangements or methodologies for providing an SDE function that allows for distinguishing the first and second strands may be utilized, such as those described in the above-referenced publications, or other methods that serves the functional purpose described.

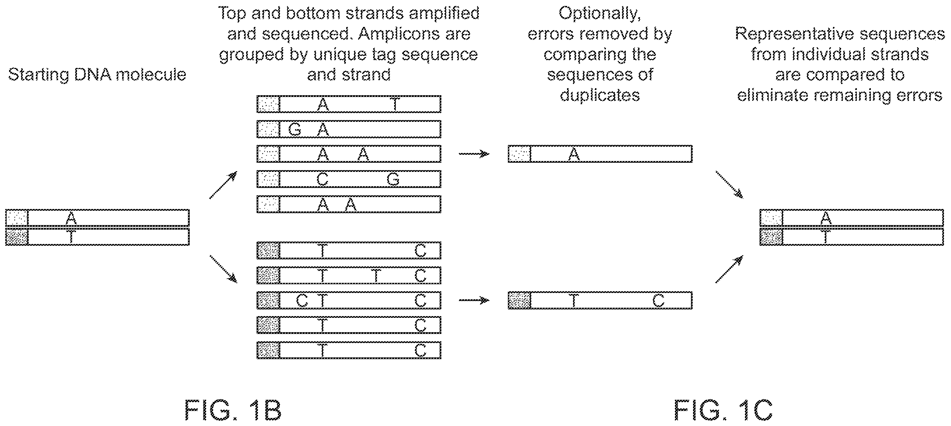

After generating the double-stranded target nucleic acid complex comprising at least one SMI and at least one SDE, or where one or both of these elements will be subsequently introduced, the complex can be subjected to DNA amplification, such as with PCR, or any other biochemical method of DNA amplification (e.g., rolling circle amplification, multiple displacement amplification, isothermal amplification, bridge amplification or surface-bound amplification, such that one or more copies of the first strand target nucleic acid sequence and one or more copies of the second strand target nucleic acid sequence are produced (e.g., FIG. **1B****)).** The one or more amplification copies of the first strand target nucleic acid molecule and the one or more amplification copies of the second target nucleic acid molecule can then be subjected to DNA sequencing, preferably using a "Next-Generation" massively parallel DNA sequencing platform (e.g., FIG. **1B****).**

In accordance with the claims, the sequence reads produced from either the first strand target nucleic acid molecule and the second strand target nucleic acid molecule, derived from the original double-stranded target nucleic acid molecule, can be identified based on sharing a related substantially unique SMI and distinguished from the opposite strand target nucleic acid molecule by virtue of an SDE. In some embodiments, the SMI may be a sequence based on a mathematically-based error correction code (for example, a Hamming code), whereby certain amplification errors, sequencing errors or SMI synthesis errors can be tolerated for the purpose of relating the sequences of the SMI sequences on complementary strands of an original Duplex (e.g., a double-stranded nucleic acid molecule). For example, with a double-stranded exogenous SMI where the SMI comprises 15 base pairs of fully degenerate sequence of canonical DNA bases, an estimated 4^15 = 1,073,741,824 SMI variants will exist in a population of the fully degenerate SMIs. If two SMIs are recovered from reads of sequencing data that differ by only one nucleotide within the SMI sequence out of a population of 10,000 sampled SMIs, one can mathematically calculate the probability of this occurring by random chance, a decision can be made whether it is more probable that the single base pair difference reflects one of the aforementioned types of errors, and the SMI sequences could be determined to have in fact derived from the same original duplex molecule. In some embodiments where the SMI is, at least in part, an exogenously applied sequence where the sequence variants are not fully degenerate to each other and are, at least in part, known sequences, the identity of the known sequences can in some embodiments be designed in such a way that one or more errors of the aforementioned types will not convert the identity of one known SMI sequence to that of another SMI sequence, such that the probability of one SMI being misinterpreted as that of another SMI is reduced. In some embodiments this SMI design strategy comprises a Hamming Code approach or derivative thereof. Once identified, one or more sequence reads produced from the first strand target nucleic acid molecule are compared with one or more sequence reads produced from the second strand target nucleic acid molecule to produce an error-corrected target nucleic acid molecule sequence (e.g., FIG. **1C****).** For example, nucleotide positions where the bases from both the first and second strand target nucleic acid sequences agree are deemed to be true sequences, whereas nucleotide positions that disagree between the two strands are recognized as potential sites of technical errors that may be discounted, eliminated, corrected or otherwise identified. An error-corrected sequence of the original double-stranded target nucleic acid molecule can thus be produced (shown in FIG. **1C****).** In some embodiments, and following separately grouping of each of the sequencing reads produced from the first strand target nucleic acid molecule and the second strand target nucleic acid molecule, a single-strand consensus sequence can be generated for each of the first and second strands. The single-stranded consensus sequences from the first strand target nucleic acid molecule and the second strand target nucleic acid molecule can then be compared to produce an error-corrected target nucleic acid molecule sequence (e.g., FIG. **1C****).**

Alternatively, in some embodiments in accordance with the claims, sites of sequence disagreement between the two strands can be recognized as potential sites of biologically-derived mismatches in the original double stranded target nucleic acid molecule. Alternatively, in some embodiments, sites of sequence disagreement between the two strands can be recognized as potential sites of DNA synthesis-derived mismatches in the original double stranded target nucleic acid molecule. Alternatively, in some embodiments, sites of sequence disagreement between the two strands can be recognized as potential sites where a damaged or modified nucleotide base was present on one or both strands and was converted to a mismatch by an enzymatic process (for example a DNA polymerase, a DNA glycosylase or another nucleic acid modifying enzyme or chemical process). In some embodiments, this latter finding can be used to infer the presence of nucleic acid damage or nucleotide modification prior to the enzymatic process or chemical treatment.

In some embodiments, and in accordance with the claims, sequencing reads generated from the Duplex Sequencing steps discussed herein can be further filtered to eliminate sequencing reads from DNA-damaged molecules (e.g., damaged during storage, shipping, during or following tissue or blood extraction, during or following library preparation, etc.). For example, DNA repair enzymes, such as Uracil-DNA Glycosylase (UDG), Formamidopyrimidine DNA glycosylase (FPG), and 8-oxoguanine DNA glycosylase (OGG1), can be utilized to eliminate or correct DNA damage (e.g., *in vitro* DNA damage or *in vivo* damage). These DNA repair enzymes, for example, are glycoslyases that remove damaged bases from DNA. For example, UDG removes uracil that results from cytosine deamination (caused by spontaneous hydrolysis of cytosine) and FPG removes 8-oxo-guanine (e.g., a common DNA lesion that results from reactive oxygen species). FPG also has lyase activity that can generate a 1 base gap at abasic sites. Such abasic sites will generally subsequently fail to amplify by PCR, for example, because the polymerase fails to copy the template. Accordingly, the use of such DNA damage repair/elimination enzymes can effectively remove damaged DNA that doesn't have a true mutation but might otherwise be undetected as an error following sequencing and duplex sequence analysis. Although an error due to a damaged base can often be corrected by Duplex Sequencing, in rare cases a complementary error could theoretically occur at the same position on both strands; thus, reducing error-increasing damage can reduce the probability of artifacts. Furthermore, during library preparation, certain fragments of DNA to be sequenced may be single-stranded from their source or from processing steps (for example, mechanical DNA shearing). These regions are typically converted to double stranded DNA during an "end repair" step known in the art, whereby a DNA polymerase and nucleoside substrates are added to a DNA sample to extend 5' recessed ends. A mutagenic site of DNA damage in the single-stranded portion of the DNA being copied (i.e. single-stranded 5' overhang at one or both ends of the DNA duplex or internal single-stranded nicks or gaps) can cause an error during the fill-in reaction that could render a single-stranded mutation, synthesis error, or site of nucleic acid damage into a double-stranded form that could be misinterpreted in the final duplex consensus sequence as a true mutation whereby the true mutation was present in the original double stranded nucleic acid molecule, when, in fact, it was not. This scenario, termed "pseudo-duplex", can be reduced or prevented by use of such damage destroying/repair enzymes. In other embodiments, this occurrence can be reduced or eliminated through use of strategies to destroy or prevent single-stranded portions of the original duplex molecule to form (e.g. use of certain enzymes being used to fragment the original double stranded nucleic acid material rather than mechanical shearing or certain other enzymes that may leave nicks or gaps). In other embodiments, use of processes to eliminate single-stranded portions of original double-stranded nucleic acids (e.g. single-stand specific nucleases such as S1 nuclease or mung bean nuclease) can be utilized for a similar purpose.

In further embodiments and in accordance with the claims, sequencing reads generated from the Duplex Sequencing steps discussed herein can be further filtered to eliminate false mutations by trimming ends of the reads most prone to pseudoduplex artifacts. For example, DNA fragmentation can generate single strand portions at the terminal ends of double-stranded molecule. These single-stranded portions can be filled in (e.g., by a polymerase) during end repair. In some instances, polymerases make copy mistakes in these end repaired regions leading to the generation of "pseudoduplex molecules." These artifacts of library preparation can incorrectly appear to be true mutations once sequenced. These errors, as a result of end repair mechanisms, can be eliminated or reduced from analysis post-sequencing by trimming the ends of the sequencing reads to exclude any mutations that may have occurred in higher risk regions, thereby reducing the number of false mutations. In one embodiment, such trimming of sequencing reads can be accomplished automatically (e.g., a normal process step). In another embodiment, a mutant frequency can be assessed for fragment end regions, and if a threshold level of mutations is observed in the fragment end regions, sequencing read trimming can be performed before generating a double-strand consensus sequence read of the DNA fragments.

By way of specific example, in some embodiments and in accordance with the claims, provided herein are methods of generating an error-corrected sequence read of a double-stranded target nucleic acid material, including the step of ligating a double-stranded target nucleic acid material to at least one adapter sequence, to form an adapter-target nucleic acid material complex, wherein the at least one adapter sequence comprises (a) a degenerate or semi-degenerate single molecule identifier (SMI) sequence that uniquely labels each molecule of the double-stranded target nucleic acid material, and (b) a first nucleotide adapter sequence that tags a first strand of the adapter-target nucleic acid material complex, and a second nucleotide adapter sequence that is at least partially non-complimentary to the first nucleotide sequence that tags a second strand of the adapter-target nucleic acid material complex such that each strand of the adapter-target nucleic acid material complex has a distinctly identifiable nucleotide sequence relative to its complementary strand. The method can next include the steps of amplifying each strand of the adapter-target nucleic acid material complex to produce a plurality of first strand adapter-target nucleic acid complex amplicons and a plurality of second strand adapter-target nucleic acid complex amplicons. The method can further include the steps of amplifying both the first and second strands to provide a first nucleic acid product and a second nucleic acid product. The method may also include the steps of sequencing each of the first nucleic acid product and second nucleic acid product to produce a plurality of first strand sequence reads and plurality of second strand sequence reads, and confirming the presence of at least one first strand sequence read and at least one second strand sequence read. The method may further include comparing the at least one first strand sequence read with the at least one second strand sequence read, and generating an error-corrected sequence read of the double-stranded target nucleic acid material by discounting nucleotide positions that do not agree, or alternatively removing compared first and second strand sequence reads having one or more nucleotide positions where the compared first and second strand sequence reads are non-complementary.

By way of an additional specific example, in some embodiments and in accordance with the claims, provided herein are methods of identifying a DNA variant from a sample including the steps of ligating both strands of a nucleic acid material (e.g., a double-stranded target DNA molecule) to at least one asymmetric adapter molecule to form an adapter-target nucleic acid material complex having a first nucleotide sequence associated with a first strand of a double-stranded target DNA molecule (e.g., a top strand) and a second nucleotide sequence that is at least partially non-complementary to the first nucleotide sequence associated with a second strand of the double-stranded target DNA molecule (e.g., a bottom strand), and amplifying each strand of the adapter-target nucleic acid material, resulting in each strand generating a distinct, yet related, set of amplified adapter-target nucleic acid products. The method can further include the steps of sequencing each of a plurality of first strand adapter-target nucleic acid products and a plurality of second strand adapter-target nucleic acid products, confirming the presence of at least one amplified sequence read from each strand of the adapter-target nucleic acid material complex, and comparing the at least one amplified sequence read obtained from the first strand with the at least one amplified sequence read obtained from the second strand to form a consensus sequence read of the nucleic acid material (e.g., a double-stranded target DNA molecule) having only nucleotide bases at which the sequence of both strands of the nucleic acid material (e.g., a double-stranded target DNA molecule) are in agreement, such that a variant occurring at a particular position in the consensus sequence read (e.g., as compared to a reference sequence) is identified as a true DNA variant.

In some embodiments and in accordance with the claims, provided herein are methods of generating a high accuracy consensus sequence from a double-stranded nucleic acid material, including the steps of tagging individual duplex DNA molecules with an adapter molecule to form tagged DNA material, wherein each adapter molecule comprises (a) a degenerate or semi-degenerate single molecule identifier (SMI) that uniquely labels the duplex DNA molecule, and (b) first and second non-complementary nucleotide adapter sequences that distinguishes an original top strand from an original bottom strand of each individual DNA molecule within the tagged DNA material, for each tagged DNA molecule, and generating a set of duplicates of the original top strand of the tagged DNA molecule and a set of duplicates of the original bottom strand of the tagged DNA molecule to form amplified DNA material. The method can further include the steps of creating a first single strand consensus sequence (SSCS) from the duplicates of the original top strand and a second single strand consensus sequence (SSCS) from the duplicates of the original bottom strand, comparing the first SSCS of the original top strand to the second SSCS of the original bottom strand, and generating a high-accuracy consensus sequence having only nucleotide bases at which the sequence of both the first SSCS of the original top strand and the second SSCS of the original bottom strand are complimentary.

In further embodiments and in accordance with the claims, provided herein are methods of detecting and/or quantifying DNA mutations and/or variants from a plurality of pooled samples comprising double-stranded target DNA molecules including the steps of ligating both strands of each double-stranded target DNA molecule to at least one asymmetric adapter molecule to form a plurality of adapter-target DNA complexes, wherein each adapter-target DNA complex has a first nucleotide sequence associated with a first strand of a double-stranded target DNA molecule and a second nucleotide sequence that is at least partially non-complementary to the first nucleotide sequence associated with a second strand of the double-stranded target DNA molecule, and for each adapter target DNA complex: amplifying each strand of the adapter-target DNA complex, resulting in each strand generating a distinct, yet related, set of amplified adapter-target DNA amplicons. The method can further include the steps of sequencing each of a plurality of first strand adapter-target DNA amplicons and a plurality of second strand adapter-target DNA amplicons, confirming the presence of at least one sequence read from each strand of the adapter-target DNA complex, and comparing the at least one sequence read obtained from the first strand with the at least one sequence read obtained from the second strand to detect and/or quantify nucleotide bases at which the sequence read of one strand of the double-stranded DNA molecule is in disagreement (e.g., non-complimentary) with the sequence read of the other strand of the double-stranded DNA molecule, such that site(s) of DNA damage can be detected and/or quantified. In some embodiments, the method can further include the steps of creating a first single strand consensus sequence (SSCS) from the first strand adapter-target DNA amplicons and a second single strand consensus sequence (SSCS) from the second strand adapter-target DNA amplicons, comparing the first SSCS of the original first strand to the second SSCS of the original second strand, and identifying nucleotide bases at which the sequence of the first SSCS and the second SSCS are non-complementary to detect and/or quantify DNA damage associated with the double-stranded target DNA molecules in the sample.

### Single Molecule Identifier Sequences (SMIs)

In accordance with various embodiments and in accordance with the claims, provided methods and compositions include one or more SMI sequences on each strand of a nucleic acid material. The SMI can be independently carried by each of the single strands that result from a double-stranded nucleic acid molecule such that the derivative amplification products of each strand can be recognized as having come from the same original substantially unique double-stranded nucleic acid molecule after sequencing. In some embodiments, the SMI may include additional information and/or may be used in other methods for which such molecule distinguishing functionality is useful, as will be recognized by one of skill in the art. In some embodiments, an SMI element may be incorporated before, substantially simultaneously, or after adapter sequence ligation to a nucleic acid material.

In some embodiments and in accordance with the claims, an SMI sequence may include at least one degenerate or semi-degenerate nucleic acid. In other embodiments, an SMI sequence may be non-degenerate. In some embodiments, an SMI sequence may be a defined nucleotide sequence. In some embodiments, the SMI can be the sequence associated with or near a fragment end of the nucleic acid molecule (e.g., randomly or semi-randomly sheared ends of ligated nucleic acid material). In some embodiments, an exogenous sequence may be considered in conjunction with the sequence corresponding to randomly or semi-randomly sheared ends of ligated nucleic acid material (e.g., DNA) to obtain an SMI sequence capable of distinguishing, for example, single DNA molecules from one another. In some embodiments, a SMI sequence is a portion of an adapter sequence that is ligated to a double-strand nucleic acid molecule. In certain embodiments, the adapter sequence comprising a SMI sequence is double-stranded such that each strand of the double-stranded nucleic acid molecule includes an SMI following ligation to the adapter sequence. In another embodiment, the SMI sequence is single-stranded before or after ligation to a double-stranded nucleic acid molecule and a complimentary SMI sequence can be generated by extending the opposite strand with a DNA polymerase to yield a complementary double-stranded SMI sequence. In other embodiments, an SMI sequence is in a single-stranded portion of the adapter (e.g., an arm of an adapter having a Y-shape). In such embodiments, the SMI can facilitate grouping of families of sequence reads derived from an original strand of a double-stranded nucleic acid molecule, and in some instances can confer relationship between original first and second strands of a double-stranded nucleic acid molecule (e.g., all or part of the SMIs maybe relatable via look up table). In embodiments, where the first and second strands are labeled with different SMIs, the sequence reads from the two original strands may be related using one or more of an endogenous SMI (e.g., a fragment-specific feature such as sequence associated with or near a fragment end of the nucleic acid molecule), or with use of an additional molecular tag shared by the two original strands (e.g., a barcode in a double-stranded portion of the adapter), or a combination thereof. In some embodiments, each SMI sequence may include between about 1 to about 30 nucleic acids (e.g., 1, 2, 3, 4, 5, 8, 10, 12, 14, 16, 18, 20, or more degenerate or semi-degenerate nucleic acids).

In some embodiments and in accordance with the claims, a SMI is capable of being ligated to one or both of a nucleic acid material and an adapter sequence. In some embodiments, a SMI may be ligated to at least one of a T-overhang, an A-overhang, a CG-overhang, a dehydroxylated base, and a blunt end of a nucleic acid material.

In some embodiments and in accordance with the claims, a sequence of a SMI may be considered in conjunction with (or designed in accordance with) the sequence corresponding to, for example, randomly or semi-randomly sheared ends of a nucleic acid material (e.g., a ligated nucleic acid material), to obtain a SMI sequence capable of distinguishing single nucleic acid molecules from one another.

In some embodiments and in accordance with the claims, at least one SMI may be an endogenous SMI (e.g., an SMI related to a shear point (e.g., a fragment end), for example, using the shear point itself or using a defined number of nucleotides in the nucleic acid material immediately adjacent to the shear point [e.g., 2, 3, 4, 5, 6, 7, 8, 9, 10 nucleotides from the shear point]). In some embodiments, at least one SMI may be an exogenous SMI (e.g., an SMI comprising a sequence that is not found on a target nucleic acid material).

In some embodiments and in accordance with the claims, a SMI may be or comprise an imaging moiety (e.g., a fluorescent or otherwise optically detectable moiety). In some embodiments, such SMIs allow for detection and/or quantitation without the need for an amplification step.

In some embodiments and in accordance with the claims a SMI element may comprise two or more distinct SMI elements that are located at different locations on the adapter-target nucleic acid complex.

Various embodiments of SMIs for use in accordance with the claims are further disclosed in International Patent Publication No. WO2017/100441.

### Strand-Defining Element (SDE)

In some embodiments and in accordance with the claims, each strand of a double-stranded nucleic acid material may further include an element that renders the amplification products of the two single-stranded nucleic acids that form the target double-stranded nucleic acid material substantially distinguishable from each other after sequencing. In some embodiments, a SDE may be or comprise asymmetric primer sites comprised within a sequencing adapter, or, in other arrangements, sequence asymmetries may be introduced into the adapter sequences and not within the primer sequences, such that at least one position in the nucleotide sequences of a first strand target nucleic acid sequence complex and a second stand of the target nucleic acid sequence complex are different from each other following amplification and sequencing. In other embodiments, the SDE may comprise another biochemical asymmetry between the two strands that differs from the canonical nucleotide sequences A, T, C, G or U, but is converted into at least one canonical nucleotide sequence difference in the two amplified and sequenced molecules. In yet another embodiment, the SDE may be or comprise a means of physically separating the two strands before amplification, such that derivative amplification products from the first strand target nucleic acid sequence and the second strand target nucleic acid sequence are maintained in substantial physical isolation from one another for the purposes of maintaining a distinction between the two derivative amplification products. Other such arrangements or methodologies for providing an SDE function that allows for distinguishing the first and second strands may be utilized.

In some embodiments and in accordance with the claims, a SDE may be capable of forming a loop (e.g., a hairpin loop). In some embodiments, a loop may comprise at least one endonuclease recognition site. In some embodiments the target nucleic acid complex may contain an endonuclease recognition site that facilitates a cleavage event within the loop. In some embodiments, a loop may comprise a non-canonical nucleotide sequence. In some embodiments, the contained non-canonical nucleotide may be recognizable by one or more enzyme that facilitates strand cleavage. In some embodiments, the contained non-canonical nucleotide may be targeted by one or more chemical process facilitates strand cleavage in the loop. In some embodiments the loop may contain a modified nucleic acid linker that may be targeted by one or more enzymatic, chemical or physical process that facilitates strand cleavage in the loop. In some embodiments this modified linker is a photocleavable linker.

A variety of other molecular tools could serve as SMIs and SDEs. Other than shear points and DNA-based tags, single-molecule compartmentalization methods that keep paired strands in physical proximity or other non-nucleic acid tagging methods could serve the strand-relating function. Similarly, asymmetric chemical labelling of the adapter strands in a way that they can be physically separated can serve an SDE role. A recently described variation of Duplex Sequencing uses bisulfite conversion to transform naturally occurring strand asymmetries in the form of cytosine methylation into sequence differences that distinguish the two strands. Although this implementation limits the types of mutations that can be detected, the concept of capitalizing on native asymmetry is noteworthy in the context of emerging sequencing technologies that can directly detect modified nucleotides. Various embodiments of SDEs are further disclosed in International Patent Publication No. WO2017/100441.

### Adapters and Adapter Sequences

In various arrangements, adapter molecules that comprise SMIs (e.g., molecular barcodes), SDEs, primer sites, flow cell sequences and/or other features are contemplated for use with many of the embodiments disclosed herein and in accordance with the claims. In some embodiments, provided adapters may be or comprise one or more sequences complimentary or at least partially complimentary to PCR primers (e.g., primer sites) that have at least one of the following properties: 1) high target specificity; 2) capable of being multiplexed; and 3) exhibit robust and minimally biased amplification.

In some embodiments and in accordance with the claims, adapter molecules can be "Y"-shaped, "U"-shaped, "hairpin" shaped, have a bubble (e.g., a portion of sequence that is non-complimentary), or other features. In other embodiments, adapter molecules can comprise a "Y"-shape, a "U"-shaped, a "hairpin" shaped, or a bubble. Certain adapters may comprise modified or non-standard nucleotides, restriction sites, or other features for manipulation of structure or function *in vitro.* Adapter molecules may ligate to a variety of nucleic acid material having a terminal end. For example, adapter molecules can be suited to ligate to a T-overhang, an A-overhang, a CG-overhang, a multiple nucleotide overhang, a dehydroxylated base, a blunt end of a nucleic acid material and the end of a molecule where the 5' of the target is dephosphorylated or otherwise blocked from traditional ligation. In other embodiments the adapter molecule can contain a dephosphorylated or otherwise ligation-preventing modification on the 5' strand at the ligation site. In the latter two embodiments, such strategies may be useful for preventing dimerization of library fragments or adapter molecules.

An adapter sequence can mean a single-strand sequence, a double-strand sequence, a complimentary sequence, a non-complimentary sequence, a partial complimentary sequence, an asymmetric sequence, a primer binding sequence, a flow-cell sequence, a ligation sequence, or other sequence provided by an adapter molecule. In particular embodiments, an adapter sequence can mean a sequence used for amplification by way of compliment to an oligonucleotide.

In some embodiments and in accordance with the claims, provided methods and compositions include at least one adapter sequence (e.g., two adapter sequences, one on each of the 5' and 3' ends of a nucleic acid material). In some embodiments, provided methods and compositions may comprise 2 or more adapter sequences (e.g., 3, 4, 5, 6, 7, 8, 9, 10 or more). In some embodiments, at least two of the adapter sequences differ from one another (e.g., by sequence). In some embodiments, each adapter sequence differs from each other adapter sequence (e.g., by sequence). In some embodiments, at least one adapter sequence is at least partially non-complementary to at least a portion of at least one other adapter sequence (e.g., is non-complementary by at least one nucleotide).

In some embodiments and in accordance with the claims, an adapter sequence comprises at least one non-standard nucleotide. In some embodiments, a non-standard nucleotide is selected from an abasic site, a uracil, tetrahydrofuran, 8-oxo-7,8-dihydro-2'deoxyadenosine (8-oxo-A), 8-oxo-7,8-dihydro-2'-deoxyguanosine (8-oxo-G), deoxyinosine, 5'nitroindole, 5-Hydroxymethyl-2' -deoxycytidine, iso-cytosine, 5 '-methyl-isocytosine, or isoguanosine, a methylated nucleotide, an RNA nucleotide, a ribose nucleotide, an 8-oxo-guanine, a photocleavable linker, a biotinylated nucleotide, a desthiobiotin nucleotide, a thiol modified nucleotide, an acrydite modified nucleotide an iso-dC, an iso dG, a 2'-O-methyl nucleotide, an inosine nucleotide Locked Nucleic Acid, a peptide nucleic acid, a 5 methyl dC, a 5-bromo deoxyuridine, a 2,6-Diaminopurine, 2-Aminopurine nucleotide, an abasic nucleotide, a 5-Nitroindole nucleotide, an adenylated nucleotide, an azide nucleotide, a digoxigenin nucleotide, an I-linker, an 5' Hexynyl modified nucleotide, an 5-Octadiynyl dU, photocleavable spacer, a non-photocleavable spacer, a click chemistry compatible modified nucleotide, and any combination thereof.

In some embodiments and in accordance with the claims, an adapter sequence comprises a moiety having a magnetic property (i.e., a magnetic moiety). In some embodiments this magnetic property is paramagnetic. In some embodiments where an adapter sequence comprises a magnetic moiety (e.g., a nucleic acid material ligated to an adapter sequence comprising a magnetic moiety), when a magnetic field is applied, an adapter sequence comprising a magnetic moiety is substantially separated from adapter sequences that do not comprise a magnetic moiety (e.g., a nucleic acid material ligated to an adapter sequence that does not comprise a magnetic moiety).

In some embodiments and in accordance with the claims, at least one adapter sequence is located 5' to a SMI. In some embodiments, at least one adapter sequence is located 3' to a SMI.

In some embodiments and in accordance with the claims, an adapter sequence may be linked to at least one of a SMI and a nucleic acid material via one or more linker domains. In some embodiments, a linker domain may be comprised of nucleotides. In some embodiments, a linker domain may include at least one modified nucleotide or non-nucleotide molecules (for example, as described elsewhere in this disclosure). In some embodiments, a linker domain may be or comprise a loop.

In some embodiments and in accordance with the claims, an adapter sequence on either or both ends of each strand of a double-stranded nucleic acid material may further include one or more elements that provide a SDE. In some embodiments, a SDE may be or comprise asymmetric primer sites comprised within the adapter sequences.

In some embodiments and in accordance with the claims, an adapter sequence may be or comprise at least one SDE and at least one ligation domain (i.e., a domain amendable to the activity of at least one ligase, for example, a domain suitable to ligating to a nucleic acid material through the activity of a ligase). In some embodiments, from 5' to 3', an adapter sequence may be or comprise a primer binding site, a SDE, and a ligation domain.

Various methods for synthesizing Duplex Sequencing adapters have been previously described in, e.g., U.S. Patent No. 9,752,188, International Patent Publication No. WO2017/100441, and International Patent Application No. PCT/US18/59908 (filed November 8, 2018).

### Primers

In some embodiments, one or more PCR primers that have at least one of the following properties: 1) high target specificity; 2) capable of being multiplexed; and 3) exhibit robust and minimally biased amplification are contemplated for use in various embodiments in accordance with the claims. A number of prior studies and commercial products have designed primer mixtures satisfying a certain number of these criteria for conventional PCR-CE. However, it has been noted that these primer mixtures are not always optimal for use with MPS. Indeed, developing highly multiplexed primer mixtures can be a challenging and time-consuming process. Conveniently, both Illumina and Promega have recently developed multiplex compatible primer mixtures for the Illumina platform that show robust and efficient amplification of a variety of standard and non-standard STR and SNP loci. Because these kits use PCR to amplify their target regions prior to sequencing, the 5'-end of each read in paired-end sequencing data corresponds to the 5'-end of the PCR primers used to amplify the DNA. In some embodiments, provided methods and compositions include primers designed to ensure uniform amplification, which may entail varying reaction concentrations, melting temperatures, and minimizing secondary structure and intra/inter-primer interactions. Many techniques have been described for highly multiplexed primer optimization for MPS applications. In particular, these techniques are often known as ampliseq methods, as well described in the art.

### Amplification

Provided methods and compositions, in various embodiments and in accordance with the claims, make use of, or are of use in, at least one amplification step wherein a nucleic acid material (or portion thereof, for example, a specific target region or locus) is amplified to form an amplified nucleic acid material (e.g., some number of amplicon products).

In some embodiments and in accordance with the claims, amplifying a nucleic acid material includes a step of amplifying nucleic acid material derived from each of a first and second nucleic acid strand from an original double-stranded nucleic acid material using at least one single-stranded oligonucleotide at least partially complementary to a sequence present in a first adapter sequence such that a SMI sequence is at least partially maintained. An amplification step further includes employing a second single-stranded oligonucleotide to amplify each strand of interest, and such second single-stranded oligonucleotide can be (a) at least partially complementary to a target sequence of interest, or (b) at least partially complementary to a sequence present in a second adapter sequence such that the at least one single-stranded oligonucleotide and a second single-stranded oligonucleotide are oriented in a manner to effectively amplify the nucleic acid material.

In some embodiments and in accordance with the claims, amplifying nucleic acid material in a sample can include amplifying nucleic acid material in "tubes" (e.g., PCR tubes), in emulsion droplets, microchambers, and other examples described above or other known vessels.

In some embodiments and in accordance with the claims, at least one amplifying step includes at least one primer that is or comprises at least one non-standard nucleotide. In some embodiments, a non-standard nucleotide is selected from a uracil, a methylated nucleotide, an RNA nucleotide, a ribose nucleotide, an 8-oxo-guanine, a biotinylated nucleotide, a locked nucleic acid, a peptide nucleic acid, a high-Tm nucleic acid variant, an allele discriminating nucleic acid variant, any other nucleotide or linker variant described elsewhere herein and any combination thereof.

While any application-appropriate amplification reaction is contemplated as compatible with some embodiments, by way of specific example, in some embodiments, an amplification step may be or comprise a polymerase chain reaction (PCR), rolling circle amplification (RCA), multiple displacement amplification (MDA), isothermal amplification, polony amplification within an emulsion, bridge amplification on a surface, the surface of a bead or within a hydrogel, and any combination thereof.

In some embodiments and in accordance with the claims, amplifying a nucleic acid material includes use of single-stranded oligonucleotides at least partially complementary to regions of the adapter sequences on the 5' and 3' ends of each strand of the nucleic acid material. In some embodiments, amplifying a nucleic acid material includes use of at least one single-stranded oligonucleotide at least partially complementary to a target region or a target sequence of interest (e.g., a genomic sequence, a mitochondrial sequence, a plasmid sequence, a synthetically produced target nucleic acid, etc.) and a single-stranded oligonucleotide at least partially complementary to a region of the adapter sequence (e.g., a primer site).

In general, robust amplification (for example PCR amplification), can be highly dependent on the reaction conditions. Multiplex PCR, for example, can be sensitive to buffer composition, monovalent or divalent cation concentration, detergent concentration, crowding agent (i.e. PEG, glycerol, etc.) concentration, primer concentrations, primer Tms, primer designs, primer GC content, primer modified nucleotide properties, and cycling conditions (*i.e.* temperature and extension times and rate of temperature changes). Optimization of buffer conditions can be a difficult and time-consuming process. In some embodiments, an amplification reaction may use at least one of a buffer, primer pool concentration, and PCR conditions in accordance with a previously known amplification protocol. In some embodiments, a new amplification protocol may be created, and/or an amplification reaction optimization may be used. By way of specific example, in some embodiments, a PCR optimization kit may be used, such as a PCR Optimization Kit from Promega^{®}, which contains a number of pre-formulated buffers that are partially optimized for a variety of PCR applications, such as multiplex, real-time, GC-rich, and inhibitor-resistant amplifications. These pre-formulated buffers can be rapidly supplemented with different Mg²⁺ and primer concentrations, as well as primer pool ratios. In addition, in some embodiments, a variety of cycling conditions (e.g., thermal cycling) may be assessed and/or used. In assessing whether or not a particular embodiment is appropriate for a particular desired application, one or more of specificity, allele coverage ratio for heterozygous loci, interlocus balance, and depth, among other aspects, may be assessed. Measurements of amplification success may include DNA sequencing of the products, evaluation of products by gel or capillary electrophoresis or HPLC or other size separation methods followed by fragment visualization, melt curve analysis using double-stranded nucleic acid binding dyes or fluorescent probes, mass spectrometry or other methods known in the art.

In accordance with various embodiments and in accordance with the claims, any of a variety of factors may influence the length of a particular amplification step (e.g., the number of cycles in a PCR reaction, etc.). For example, in some embodiments, a provided nucleic acid material may be compromised or otherwise suboptimal (e.g. degraded and/or contaminated). In such case, a longer amplification step may be helpful in ensuring a desired product is amplified to an acceptable degree. In some embodiments, an amplification step may provide an average of 3 to 10 sequenced PCR copies from each starting DNA molecule, though in other embodiments, only a single copy of each of a first strand and second strand are required. Without wishing to be held to a particular theory, it is possible that too many or too few PCR copies could result in reduced assay efficiency and, ultimately, reduced depth. Generally, the number of nucleic acid (e.g., DNA) fragments used in an amplification (e.g., PCR) reaction is a primary adjustable variable that can dictate the number of reads that share the same SMI/barcode sequence.

### Nucleic Acid Material

### Types

Any of a variety of nucleic acid material may be used. Nucleic acid material may comprise at least one modification to a polynucleotide within the canonical sugar-phosphate backbone. Nucleic acid material may comprise at least one modification within any base in the nucleic acid material. For example, by way of non-limiting example, , the nucleic acid material is or comprises at least one of double-stranded DNA, single-stranded DNA, double-stranded RNA, single-stranded RNA, peptide nucleic acids (PNAs), locked nucleic acids (LNAs).

### Modifications

In accordance with various embodiments and in accordance with the claims, nucleic acid material may receive one or more modifications prior to, substantially simultaneously, or subsequent to, any particular step, depending upon the application for which a particular provided method or composition is used.

In some embodiments and in accordance with the claims, a modification may be or comprise repair of at least a portion of the nucleic acid material. While any application-appropriate manner of nucleic acid repair is contemplated as compatible with some embodiments, certain exemplary methods and compositions therefore are described below and in the Examples.

By way of non-limiting example, in some embodiments and in accordance with the claims, DNA repair enzymes, such as Uracil-DNA Glycosylase (UDG), Formamidopyrimidine DNA glycosylase (FPG), and 8-oxoguanine DNA glycosylase (OGG1), can be utilized to correct DNA damage (e.g., *in vitro* DNA damage). As discussed above, these DNA repair enzymes, for example, are glycoslyases that remove damaged bases from DNA. For example, UDG removes uracil that results from cytosine deamination (caused by spontaneous hydrolysis of cytosine) and FPG removes 8-oxo-guanine (e.g., most common DNA lesion that results from reactive oxygen species). FPG also has lyase activity that can generate 1 base gap at abasic sites. Such abasic sites will subsequently fail to amplify by PCR, for example, because the polymerase fails copy the template. Accordingly, the use of such DNA damage repair enzymes can effectively remove damaged DNA that doesn't have a true mutation but might otherwise be undetected as an error following sequencing and duplex sequence analysis.

As discussed above, in further embodiments and in accordance with the claims, sequencing reads generated from the processing steps discussed herein can be further filtered to eliminate false mutations by trimming ends of the reads most prone to artifacts. For example, DNA fragmentation can generate single-strand portions at the terminal ends of double-stranded molecules. These single-stranded portions can be filled in (e.g., by Klenow) during end repair. In some instances, polymerases make copy mistakes in these end-repaired regions leading to the generation of "pseudoduplex molecules." These artifacts can appear to be true mutations once sequenced. These errors, as a result of end repair mechanisms, can be eliminated from analysis post-sequencing by trimming the ends of the sequencing reads to exclude any mutations that may have occurred, thereby reducing the number of false mutations. In some embodiments, such trimming of sequencing reads can be accomplished automatically (e.g., a normal process step). In some embodiments, a mutant frequency can be assessed for fragment end regions and if a threshold level of mutations is observed in the fragment end regions, sequencing read trimming can be performed before generating a double-strand consensus sequence read of the DNA fragments.

The high degree of error correction provided by the strand-comparison technology of Duplex Sequencing reduces sequencing errors of double-stranded nucleic acid molecules by multiple orders of magnitude as compared with standard next-generation sequencing methods. This reduction in errors improves the accuracy of sequencing in nearly all types of sequences but can be particularly well suited to biochemically challenging sequences that are well known in the art to be particularly error prone. One non-limiting example of such type of sequence is homopolymers or other microsatellites/short-tandem repeats. Another non-limiting example of error prone sequences that benefit from Duplex Sequencing error correction are molecules that have been damaged, for example, by heating, radiation, mechanical stress, or a variety of chemical exposures which creates chemical adducts that are error prone during copying by one or more nucleotide polymerases and also those that create single-stranded DNA at ends of molecules or as nicks and gaps. In further embodiments, Duplex Sequencing can also be used for the accurate detection of minority sequence variants among a population of double-stranded nucleic acid molecules. One non-limiting example of this application is detection of a small number of variant DNA molecules (e.g., variant alleles representative of genetic disease or disorder) among a larger number of DNA molecules. For example, many patient samples can be screened for variant alleles correlating or causative of rare genetic disease. Another non-limiting application for rare variant detection by Duplex Sequencing is early detection of carriers of genetic variant alleles that could present later symptoms of associated genetic disease and/or could pass such genetic variant alleles to offspring. A further non-limiting application of Duplex Sequencing is for determination of variant allele frequency in a large patient population. Another non-limiting application of Duplex Sequencing is for genotyping a plurality biological samples for assessing the presence of disease-causing or disease-correlative genetic variants in a cost-efficient manner.

### III. Selected Embodiments of Methods for Genotyping Large Numbers of Samples Via Pooling

Many rare genetic diseases are detected in individuals only after symptoms have arisen, delaying treatment and possibly leading to irrevocable harm. Existing or to-be-developed drugs that may benefit certain subsets of these patients having homozygous or heterozygous variants in one or more genes can be administered earlier (e.g., prior to symptom onset) if such subsets of the population could be identified. As such, the problem of identifying carriers of rare disease alleles amongst large populations has relevance to screen patients for early intervention as well as for drug development for novel biomarkers/candidates. Additionally, identifying carriers of rare disease alleles can be used to inform patients and health care providers regarding risks associated with passing such disease alleles to off-spring (e.g., for purposes of genetic counseling, etc.).

Using conventional NGS-based sequencing methods, screening for rare genetic variants results in high monetary costs associated with preparing each sample individually and for sequencing such samples. For example, each sample would have to be individually index barcoded before pooling the samples for multiplex sequencing. As such, the cost of library preparation and sequencing each sample scales linearly with the number of samples making screening large numbers (e.g., patient populations, racial populations, etc.) a cost-ineffective tool. However, in embodiments in accordance with the present technology, the cost of sample prep rises less than linearly with the number of samples.

The present disclosure provides methods for screening and genotyping large numbers of nucleic acid samples using next generation DNA sequencing and multiplexing tools for distinguishing sequencing data for each sample are disclosed herein and are in accordance with the claims. In one embodiment, the method can identify relatively infrequently occurring variants among a plurality of samples by sequencing unindexed mixtures of the original nucleic acid samples.

In some embodiments, provided methods are useful for genotyping a plurality of biological samples via pooling samples into sub-pooled sample mixtures which can be prepared and sequenced in accordance with the claims. In some embodiments, provided methods are useful for screening biological sources for a genetic variant. In some embodiments, the genetic variant is associated with a rare disease. In some embodiments, provided methods are useful for identifying a subject having a rare variant allele among a population of subjects. In some embodiments, provided methods are useful for screening patient DNA samples for rare variant allele(s).

The method for genotyping a plurality of biological samples in accordance with aspects of the present technology, includes a step of pooling the plurality of biological samples into a unique combination of sub-pools, wherein each biological sample comprises target double-stranded DNA molecules. In one embodiment, one or more of the biological samples can be derived from different subjects. Double-stranded DNA molecules can be extracted from samples derived from the subjects (e.g., tissue samples, blood samples, etc.). In some embodiments, double-stranded DNA molecules can be isolated from non-cellular DNA, such as cell-free DNA or DNA from exosomes or other extracellular vesicles from a subject. Genotyping and/or screening of artificial sources (e.g., synthetic oligonucleotides, gene-edited samples, manufactured cell population, manufactured viral samples, synthetic nucleotides providing information storage, etc.) are also contemplated.

Provided methods include generating an error-corrected sequence read for each of a plurality of the target double-stranded DNA molecules in the sub-pools. In a particular example, error-corrected sequence reads can be generated using Duplex Sequencing. Provided methods include identifying a presence of one or more variant alleles from the error-corrected sequence reads, and determining the original biological sample containing the variant allele(s). In some embodiments, the original biological sample containing the variant allele (e.g., prior to pooling) can be determined by identifying the unique combination of sub-pools containing the variant allele(s).

Using the digital nature of NGS, aspects of the present technology are able to resolve subclonal mixtures. For example, if relatively equal parts of DNA purified from 10 subjects were combined and one subject carried a heterozygous mutation for a particular gene that the other nine individuals did not and this was sequenced to a sufficient depth (i.e. number of redundant genomic copies), one would expect to find this variant in ½ * 10 = 1/20 = 5% of the molecular copies of this gene. In this example it would be possible to mix the DNA of ten subjects together, prepare a single sequencing library and genotype this mixture to infer the presence of one sample. In this example because one knows the number of individuals mixed and the inputted DNA from each, the variant allele fraction itself indicates how many individuals carried the mutation. Examples of methods for resolving nucleic acid mixtures is described in International Patent Application No. PCT/US2019/032755.

In examples in which a subject having a variant allele cannot be known *a priori,* aspects of the present technology provide methods for screening and/or genotyping a plurality of biological samples (e.g., a large number of samples, samples from multiple subjects, etc.) by pooling the samples in unique combinations of sub-pools of samples in accordance with the claims. For example, individual biological samples (e.g., nucleic acid samples derived from an original source/subject/patient, etc.) can be aliquoted or subdivided (e.g., evenly or unevenly) into a plurality of sub-pooled sample mixtures. In certain embodiments, each individual biological sample is aliquoted into a unique combination of sub-pooled sample mixtures, such that no two individual biological samples are aliquoted into the same combination of sub-pooled sample mixtures. Hence, in some embodiments, the unique combination of sub-pooled sample mixtures can serve as a unique sample identifier. In one embodiment, nucleic acid molecules originating from each individual biological sample need not be tagged with an exogenous indexing barcode prior to aliquoting the individual biological samples. In one embodiment, the sub-pooled sample mixtures are tagged with an indexing barcode such that sequence reads derived from nucleic acid molecules in each particular sub-pooled sample mixture can be distinguished from sequence reads derived from nucleic acid molecules in the other sub-pooled sample mixtures. In embodiments, the number of individual biological samples exceeds the number of sub-pooled sample mixtures.

In some embodiments and in accordance with the claims, multiple sub-pooled sample mixtures containing different combinations of individual biological samples can be analyzed via Duplex Sequencing for the presence of a variant allele (e.g., a genetic mutation, a disease-associated allele, etc.). Sequencing reads revealing a particular variant allele in a particular combination of sub-pooled sample mixtures will allow inference of the contributor.

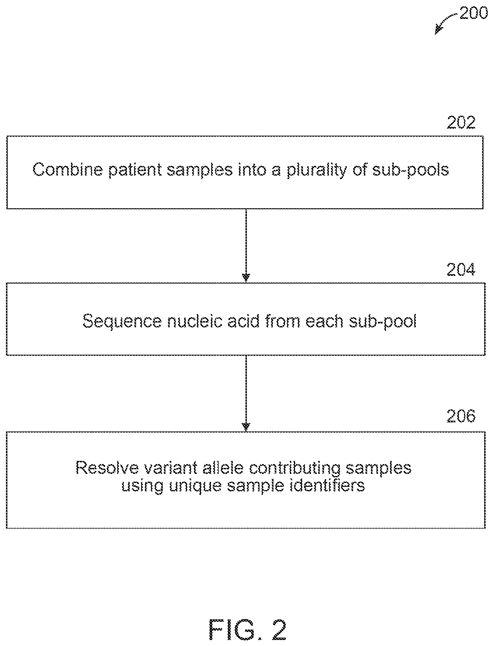

An embodiment of a method for efficient genotyping of a large number of samples is illustrated in FIG. **2** and in accordance with the claims. As shown in FIG. **2****,** and in a first step of the method **200,** a large number of patient samples are pooled into a smaller number of pooled DNA samples (block **202).** In this step, each individual nucleic acid (e.g., DNA) sample will be aliquoted into a small and unique subset of the DNA pools, e.g. into 4 of 30 available pools. The unique subset of pools into which each individual sample is aliquoted forms a unique sample identifier (e.g., a "barcode") for that sample (e.g., a particular patient sample). The method **200** also includes assaying all the sample pools with Duplex Sequencing over the relevant genomic region(s) (e.g., regions of sequencing inquiry) (step **204).** If a patient has a variant allele (e.g., an allele or mutation associated with a disease), then that allele will be sequenced and represented in the sequence reads generated in each of the pools in that sample's unique subset of pools (e.g., as identified by the assigned unique sample identifier). The method **200** further includes, resolving variant allele contributing samples by querying the matching unique sample identifiers (step **206).**

In another embodiment and in accordance with the claims, methods as described herein provide the variant allele frequency (VAF) for each variant allele present in the sub-pooled sample mixtures (e.g., also referred to herein as "sub-pools"), which can used to determine whether an individual subject is homozygous or heterozygous for a particular genetic variant (e.g., a disease-associated mutation). In one non-limiting example, if ten samples are aliquoted into 5 defined sub-pools, each sub-pool having 5 aliquoted samples each, where the contributors to each pool were different, the presence of 1/5 x ½ = 10% VAF in each sub-pool within a unique combination of sub-pools, would indicate the presence of a heterozygous mutant individual subject within the population of individual subjects screened. The unique combination of sub-pools identified as having 10% VAF of the particular genetic variant is used as the unique sample identifier (e.g., an aliquot pattern established during the sub-pool sample mixing steps) to infer the specific individual subject. It will be apparent to one experienced in the art that various mixing schemes could be applied such that the pattern of sub-pools (e.g., plate wells, tubes, mixing vessels, etc.) with the variant allele/mutation found could narrow down the number of possible individuals who carry the mutation to exactly one, or a subset of the ten.

In some examples, the number sub-pools generated and the number of individual biological samples represented in each sub-pool is such that it is possible to create an "edit distance" between their patterns such that an error due to a non-recovered mutation or a false mutation would not irreversibly obscure the individual contributing this mutation (i.e. the pattern tolerates some errors). Similarly, if a given variant is present in several samples that were pooled and a given sub-pool has contribution from multiple of these, as the rarity of a given variant decreases, the probability of this becomes higher and distinguishing can become more challenging. Such "edit distance" approaches become additionally useful here as well.

By generating error-corrected sequence reads, embodiments presented herein in accordance with the claims overcome the challenge associated with the about 1% error rate associated with standard NGS. For example, low frequency (e.g., rare) variant alleles in mixtures of 100 or more individuals, would be obscured by artefactual mutations that arise and distort the signal (e.g., error noise obscures the detection of the true variant alleles) when using standard NGS sequencing. Methods providing high accuracy sequencing reads, such as Duplex Sequencing, as described herein and elsewhere, can reduce or eliminate these errors to allow mixing of hundreds or thousands of samples and still be able to confidently detect an originating source with high sensitivity and specificity.

Various embodiments in accordance with the claims comprising a sub-pooling approach for providing a unique sample identifier without individually index barcoding nucleic acid molecules in each individual sample might have a more minimal cost saving when screening and/or genotyping a number of samples below a cost-savings threshold (e.g., 10, 20, 30, etc.); however when screening and/or genotyping hundreds or thousands of samples, the cost and time savings associated with actual library preparation of the sub-pooled sample mixtures would increase dramatically. Table 1 shows a variety of unique sample identifiers (i.e., pool "barcode" schemes), along with pertinent performance characteristics.

**Table 1**

| Number of sub-pools/ barcode | Number of sub-pools | Number of unique IDs with edit distance > 1 | Max samples such that average sub-pool has <200 samples | Number of sample preps | Number of sample preps per sample |
|---|---|---|---|---|---|
| 4 | 15 | 81 | 81 | 15 | 0.19 |
| 4 | 17 | 126 | 126 | 17 | 0.13 |
| 4 | 20 | 213 | 213 | 20 | 0.09 |
| 4 | 22 | 284 | 284 | 22 | 0.08 |
| 4 | 25 | 440 | 440 | 25 | 0.06 |
| 4 | 27 | 563 | 563 | 27 | 0.05 |
| 4 | 30 | 798 | 798 | 30 | 0.04 |
| 4 | 32 | 977 | 977 | 32 | 0.03 |
| 4 | 35 | 1299 | 1299 | 35 | 0.03 |
| 4 | 37 | 1537 | 1537 | 37 | 0.02 |
| 4 | 40 | 1985 | 1985 | 40 | 0.02 |
| 4 | 42 | 2303 | 2100 | 42 | 0.02 |
| 4 | 45 | 2862 | 2250 | 45 | 0.02 |
| 4 | 47 | 3295 | 2350 | 47 | 0.02 |
| 4 | 50 | 3979 | 2500 | 50 | 0.02 |
| 4 | 55 | 5379 | 2750 | 55 | 0.02 |
| 4 | 57 | 6021 | 2850 | 57 | 0.02 |
| 4 | 60 | 7054 | 3000 | 60 | 0.02 |
| 4 | 62 | 7819 | 3100 | 62 | 0.02 |

For example, as the number of generated sub-pooled sample mixtures increases (Table 1, second column), a number of individual biological samples that can be uniquely identified (when aliquoting each sample into 4 sub-pools) rises non-linearly (see third column). If maintaining an average number of <200 samples per sub-pool, the number of sample preps/sample (see sixth column) declines rapidly when increasing the number of sub-pools from 15 to about 40. As the cost of sequencing each individual sample scales linearly with the number of samples to be prepared and sequenced, when using a sub-pool sample mixing scheme, the costs associated with sequencing library preparation and sequencing per sample decline. Said another way, in embodiments in accordance with the present technology, the cost of sample prep rises less than linearly with an increase in number of individual samples.

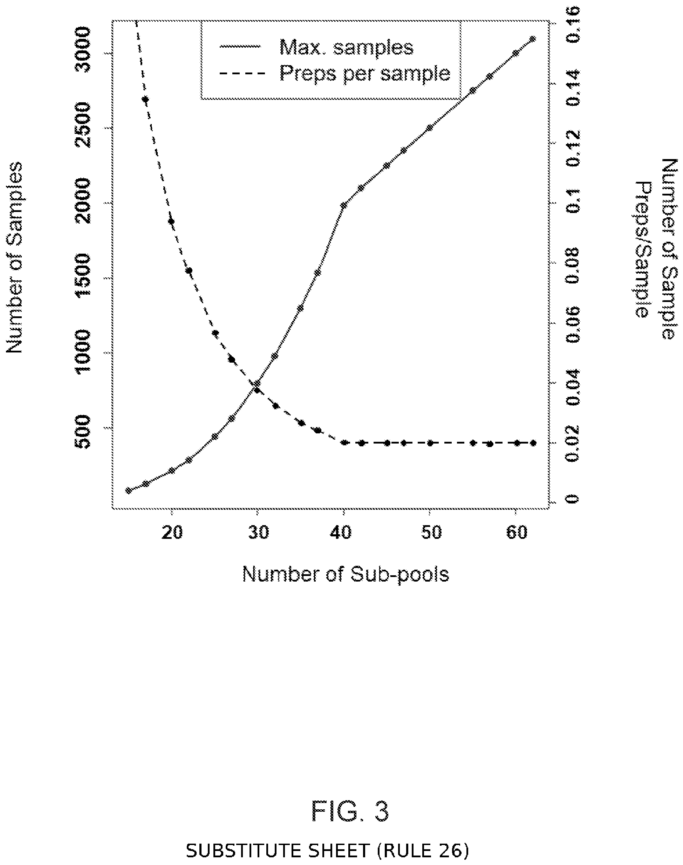

FIG. **3** is a plot showing cost and performance metrics for sample pooling schemes in accordance with an embodiment of the present technology and in accordance with the claims . As a non-limiting example, FIG. **3** illustrates a pooling scheme that assigns 4 sub-pools to each individual sample while requiring an edit distance of at least 2 between unique sample identifiers (e.g., at least 2 pools are different between each sample sub-pool combination). It will be appreciated that a variety of edit distances can be used in addition to a number of sub-pools a sample is assigned (e.g., varying the length of a sample identifier or code).

In an example, a Hamming code or any other published or unpublished error-correcting code can be used to determine a unique sample identifier with sufficient edit distance. In some examples, unique sample identifiers that have an "edit distance" of at least 2 from each other. For example, there must be a mis-identified genotype in at least two sub-pools to call an incorrect sample as a carrier of a particular genetic variant, or to be unable to distinguish which of two (2) individuals' samples is a carrier. In another example, an edit distance as low as 1 or higher than two could also be used.

In further embodiments and in accordance with the claims, samples could be assigned to different numbers of pools, providing further distinction between unique sample identifiers. In a particular example illustrated in Table 1, and to avoid needing to prepare any given sub-pool more than once for sequencing, one embodiment can have an average of 200 samples/pool. As shown in FIG. 3, adding more sub-pools greatly increases the number of samples that can be investigated in a single study; however, the cost of sample preparation per sample decreases sharply until around 40 pools. Accordingly, in some embodiments, the number of sub-pooled sample mixtures (i.e., sub-pools) can be 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 42, 45, 47, 50, 52, 55, 57, 60, 62, 65, 67, 70 or more. For example, the number of sub-pooled sample mixtures can between 15 and about 40, about 30 to about 50, about 35 to about 55, about 40 to about 60, or over 60 sub-pools.

In some embodiments, a method for screening biological sources for a genetic variant are provided in accordance with the claims. In a particular example, it is useful to screen large populations of individual subjects (e.g., patients) to determine if any members of the population are carriers of one or more genetic variants associated with a disease or disorder. In an embodiment, the method provides a step comprising aliquoting a plurality of biological samples derived from the biological sources into a unique combination of sub-pools, wherein each biological sample comprises target double-stranded DNA molecules. In an embodiment, each biological sample is aliquoted into more than one sub-pool such that the unique combination of sub-pools provides a sample identifier. In one embodiment, the method provides the steps of generating an error-corrected sequence read for each of a plurality of the target double-stranded DNA molecules in the sub-pools; identifying a presence of one or more variant allele(s) from the error-corrected sequence reads; and determining the biological source containing the variant allele(s) by identifying the unique combination of sub-pools containing the variant allele(s).

In some embodiments are provided methods of generating high accuracy sequencing reads of a population of target double-stranded nucleic acid molecules within each sub-pooled sample mixture (i.e., sub-pool) in accordance with the claims. Such methods include Duplex Sequencing of one or more target double-stranded nucleic acid molecules within each sub-pooled sample mixture and generating high accuracy consensus sequences for the targeted double-stranded DNA molecules. In some embodiments, target double-stranded nucleic acid molecules comprise a targeted genomic region or genetic loci associated with a disease or disorder (e.g., cancer, non-cancer disease, rare genetic disease, etc.). In some embodiments, provided methods further include comparing one or more error-corrected sequence reads comprising a sequence at a targeted genomic locus to a reference sequence.

In some embodiments and in accordance with the claims, the generation of an error-corrected sequence read for each of a plurality of the double-stranded DNA molecules further comprises selectively enriching one or more targeted genomic regions prior to sequencing to provide a plurality of enriched adapter-DNA molecules. In some embodiments, provided methods are useful for identifying one or more genetic variants among double-stranded DNA molecules within a sub-pooled sample mixture (e.g., double-stranded DNA molecules that originated and/or were extracted from a subject carrying genetic variant). In some embodiments, provided methods include a step of identifying one or more genetic variants among double-stranded DNA molecules within a sub-pooled sample mixture.

In some embodiments and in accordance with the claims, one or more targeted genomic regions to be analyzed by Duplex Sequencing methods are or include a target genomic locus in the genome. In some embodiments, provided methods include a step of determining if one or more error-corrected sequence reads comprises a rare or disease-associated genetic variant at the target genomic locus. Thus, provided methods may be useful for assessing or screening populations of subjects for carriers of particular genetic variants of interest. For example, one or more targeted genomic regions comprise genes known to harbor disease-causing mutations. In some embodiments, a disease-causing mutation is or includes a loss of function mutation, a gain of function mutation, or a dominant negative mutation. In another embodiment, one or more targeted genomic regions comprise genetic loci known to be associated with a disease or disorder. In an embodiment, the disease or disorder is a rare genetic disorder. In one embodiment, the disease or disorder is a single-gene disorder (e.g., a mutation is found in a single gene). In another embodiment, the disease or disorder is a complex disorder involving mutations in two or more genes. In one embodiment, the disease or disorder is associated with an autosomal recessive mutation. In another embodiment, the disease or disorder is associated with an autosomal dominant mutation.

In some embodiments and in accordance with the claims, provided herein are methods for efficiently screening populations of subjects (e.g., hundreds or thousands of subjects). In some embodiments, provided methods comprise a step of determining a frequency of the genetic variant among the error-corrected sequence reads comprising the sequence at the target genomic locus. In some embodiments provided herein are methods for determining if a carrier of a genetic variant is homozygous or heterozygous for the genetic variant. In some embodiments provided herein are methods for determining a frequency of the genetic variant among a population, e.g., frequency of carriers of a particular genetic variant in the represented population.

In some embodiments and in accordance with the claims, provided methods include analyzing the one or more correspondences between first and second strand sequence reads derived from double-stranded DNA molecules comprising sequences from one or more target genomic loci; and comparing the correspondences to a reference genome sequence; and determining the frequency of the one or more variants among the plurality of double-stranded DNA molecules comprising the one or more target genomic loci. In some embodiments, provided herein are methods for genotyping and/or screening for genetic variants at one or more target genomic loci (e.g., 1, 2, 3, 4, 5, 6, 7, 8, 9, 10 or more).

In some embodiments and in accordance with the claims, a target genomic locus is or comprises a tumor suppressor gene, an oncogene, a proto-oncogene, and/or a cancer driver. In some embodiments, a cancer driver is a known cancer driver from Cancer Gene Census (CGC) or the COSMIC database (genes causally implicated in cancer). In some embodiments, a cancer driver gene is or includes: *ABL, ACC, BCR, BLCA, BRCA, CESC, CHOL, COAD, DLBC, DNMT3A, EGFR, ESCA, GBM, HNSC, KICH, KIRC, KIRP, LAML, LGG, LIHC, LUAD, LUSC, MESO, OV, PAAD, PCPG, PI3K, PIK3CA, PRAD, PTEN, RAS, READ, SARC, SKCM, STAD, TGCT, THCA, THYM, TP53, UCEC, UCS,* and/or *UVM.* In some embodiments, if a variant is detected in one or more cancer drivers among the plurality of enriched tagged DNA molecules in the population of molecules, then the method may further include a step of determining a variant frequency of the variant among the plurality of enriched tagged DNA molecules.

In some embodiments and in accordance with the claims, a target genomic region is or comprises known genes or loci associated with rare genetic disorders or disease. In some embodiments, methods are provided for screening human patients for a rare genetic disorder or disease. Some non-limiting examples of a rare genetic disorder or disease include phenylketonuria (PKU), cystic fibrosis, sickle-cell anemia, some forms of albinism, Huntington's disease, myotonic dystrophy type 1, familial hypercholesterolemia, neurofibromatosis, polycystic kidney disease 1 and 2, hemophilia A and B, muscular dystrophy (Duchenne type), hypophosphatemic rickets, Rett's syndrome, Tay-Sachs disease, Wilson disease, Werner syndrome, fatal familial insomnia and/or genetic forms of spermatogenic failure. In some embodiments, rare genetic disorders or diseases and associated target genome loci is or includes those found at https://www.omim.org and

### https://www.ncbi.nlm.nih.gov/clinvar/.

In one embodiment and in accordance with the claims, a target genomic region is or comprise a genomic locus associated with a rare genetic disorder of obesity. For example, a rare genetic disorder of obesity is or includes Proopiomelanocortin (POMC) Deficiency Obesity, Alström syndrome, Leptin Receptor (LEPR) Deficiency Obesity, Prader-Willi syndrome (PWS), Bardet-Biedl syndrome (BBS), and high-impact Heterozygous Obesity.

In another embodiment and in accordance with the claims, method provided herein can be useful for high-throughput screening of non-human (e.g., non-animal) samples. In one embodiment, methods used herein can be useful for screening samples derived from plants (e.g., gene-edited plant cells, plant hybrids, designer plant species). In another embodiment, mutagenized plant cells can be screened quickly and efficiently for desired genetic modifications.

In another embodiment and in accordance with the claims, where a genetic variant is common enough to be expected to occur more than once among the individual biological samples(e.g., 2, 3, 4, 5 or more carriers), distinct haplotypes comprising the variant can be uniquely identified from other variants on the same sequencing read. In this embodiment, the genetic variant would be (a) proximal on the genome to a single-nucleotide polymorphism (SNP) and (b) not be in perfect linkage disequilibrium with the SNP. Accordingly, if two individuals share the same disease allele but differ in their genotype for a proximal SNP, they can be uniquely identified. For example, any sequencing reads that span both the disease variant and the SNP can be used to distinguish the two instances of the variant.

In a further embodiment and in accordance with the claims, wherein a risk allele is common enough to be expected to occur in multiple biological samples within a study, the variant frequency of the variant allele in a sub-pool can be used to estimate how many samples in the sub-pool carry the variant allele, thereby enabling deconvolution of multiple carriers who overlap in some pools.

In some embodiments and in accordance with the claims, fewer sub-pools could be created and/or less complex unique sample identifiers (e.g., pool "barcodes") could be used. In a particular example, many individual samples could be assigned the same unique sample identifier (e.g., "non-uniquely" identifiable/labelled). Following Duplex Sequencing of the samples, a disease allele carrier is not uniquely identified but results effectively narrow down the carrier to a short list of original samples, which could be interrogated with individual sequencing schemes (e.g., Sanger sequencing, NGS, etc.). Accordingly, and in some embodiments, such methods can effectively and efficiently narrow down a very large population of samples to a more reasonable number of samples to pursue in follow-on sequencing assays.

In some embodiments and in accordance with the claims, appending demultiplexing indices to samples or to groups of samples can be used before the sub-pooling process, allowing for samples to be pooled having the same sample identifier but still be distinguishable from each other.

In one embodiment and in accordance with the claims, a large number (e.g., hundreds, thousands, etc.) of samples could be sequenced in a single pool in order to identify whether any specific disease allele was present in the pool. In one embodiment, such a step could be taken prior to sequencing sub-pools of the samples. For example, a "single pool" embodiment could then be used as a gating mechanism for running a more expensive (e.g., cost, time, etc.) study described in other embodiments herein. In particular, one may choose to run a sub-pooling study only if the single pool study shows there's a risk allele carrier to be found in the study population.

In yet a further embodiment and in accordance with the claims, sequencing costs could be reduced when each sample is aliquoted into a larger number of pools with each pool being sequenced to a lower depth. When sequencing at lower depth, some variant alleles may not be sequenced in pools in which it is present; however, with each sample represented in a greater number of sub-pools prior to sequencing, an identified variant allele can be still be identified to the original contributing sample. For example, each sample can be aliquoted into 8 sub-pools and each sub-pool is sequenced to a depth such that a variant is expected to go undetected approximately 30% of the time. In this example, unique sample identifiers with an edit distance of 4 can be used to associate most variants to a single original sample. In some embodiments, each original sample can be aliquoted into 1, 2, 3, 4, 5, 6, 7, 8, 9, 10 or more sub-pools.

### Kits with Reagents

Aspects of the present technology but not claimed further encompass kits for conducting various aspects of Duplex Sequencing methods (also referred to herein as a "DS kit"). In some examples, a kit may comprise various reagents along with instructions for conducting one or more of the methods or method steps disclosed herein for nucleic acid extraction, nucleic acid library preparation, amplification (e.g. via PCR) and sequencing. In one example, a kit may further include a computer program product (e.g., coded algorithm to run on a computer, an access code to a cloud-based server for running one or more algorithms, etc.) for analyzing sequencing data (e.g., raw sequencing data, sequencing reads, etc.) to determine, for example, a variant allele, mutation, etc., associated with a sample and in accordance with aspects of the present technology. Kits may include DNA standards and other forms of positive and negative controls.

In some examples, a DS kit may comprise reagents or combinations of reagents suitable for performing various aspects of sample preparation (e.g., tissue manipulation, DNA extraction, DNA fragmentation), nucleic acid library preparation, amplification and sequencing. For example, a DS kit may optionally comprise one or more DNA extraction reagents (e.g., buffers, columns, etc.) and/or tissue extraction reagents. Optionally, a DS kit may further comprise one or more reagents or tools for fragmenting double-stranded DNA, such as by physical means (e.g., tubes for facilitating acoustic shearing or sonication, nebulizer unit, etc.) or enzymatic means (e.g., enzymes for random or semi-random genomic shearing and appropriate reaction enzymes). For example, a kit may include DNA fragmentation reagents for enzymatically fragmenting double-stranded DNA that includes one or more of enzymes for targeted digestion (e.g., restriction endonucleases, CRISPR/Cas endonuclease(s) and RNA guides, and/or other endonucleases), double-stranded Fragmentase cocktails, single-stranded DNase enzymes (e.g., mung bean nuclease, S1 nuclease) for rendering fragments of DNA predominantly double-stranded and/or destroying single-stranded DNA, and appropriate buffers and solutions to facilitate such enzymatic reactions.

In an example, a DS kit comprises primers and adapters for preparing a nucleic acid sequence library from a sample that is suitable for performing Duplex Sequencing process steps to generate error-corrected (e.g., high accuracy) sequences of double-stranded nucleic acid molecules in the sample. For example, the kit may comprise at least one pool of adapter molecules comprising single molecule identifier (SMI) sequences or the tools (e.g., single-stranded oligonucleotides) for the user to create it. In some examples, the pool of adapter molecules will comprise a suitable number of substantially unique SMI sequences such that a plurality of nucleic acid molecules in a sample can be substantially uniquely labeled following attachment of the adapter molecules, either alone or in combination with unique features of the fragments to which they are ligated. One experienced in the art of molecular tagging will recognize that what entails a "suitable" number of SMI sequences will vary by multiple orders of magnitude depending on various specific factors (input DNA, type of DNA fragmentation, average size of fragments, complexity vs repetitiveness of sequences being sequenced within a genome etc.) Optionally, the adaptor molecules further include one or more PCR primer binding sites, one or more sequencing primer binding sites, or both. In another example, a DS kit does not include adapter molecules comprising SMI sequences or barcodes, but instead includes conventional adapter molecules (e.g., Y-shape sequencing adapters, etc.) and various method steps can utilize endogenous SMIs to relate molecule sequence reads. In some examples, the adapter molecules are indexing adapters and/or comprise an indexing sequence. In other examples, indexes are added to specific samples through "tailing in" by PCR using primers supplied in a kit

In an example, a DS kit comprises a set of adapter molecules each having a non-complementary region and/or some other strand defining element (SDE), or the tools for the user to create it (e.g., single-stranded oligonucleotides). In another example, the kit comprises at least one set of adapter molecules wherein at least a subset of the adapter molecules each comprise at least one SMI and at least one SDE, or the tools to create them. Additional features for primers and adapters for preparing a nucleic acid sequencing library from a sample that is suitable for performing Duplex Sequencing process steps are described above as well as disclosed in U.S. Patent No. 9,752,188, International Patent Publication No. WO2017/100441, and International Patent Application No. PCT/US18/59908 (filed November 8, 2018).

Additionally, a kit may further include DNA quantification materials such as, for example, DNA binding dye such as SYBR^{™} green or SYBR^{™} gold (available from Thermo Fisher Scientific, Waltham, MA) or the alike for use with a Qubit fluorometer (e.g., available from Thermo Fisher Scientific, Waltham, MA), or PicoGreen^{™} dye (e.g., available from Thermo Fisher Scientific, Waltham, MA) for use on a suitable fluorescence spectrometer or a real-time PCR machine or digital-droplet PCR machine. Other reagents suitable for DNA quantification on other platforms are also contemplated. Further examples include kits comprising one or more of nucleic acid size selection reagents (e.g., Solid Phase Reversible Immobilization (SPRI) magnetic beads, gels, columns), columns for target DNA capture using bait/pray hybridization, qPCR reagents (e.g., for copy number determination) and/or digital droplet PCR reagents. In some examples, a kit may optionally include one or more of library preparation enzymes (ligase, polymerase(s), endonuclease(s), reverse transcriptase for e.g., RNA interrogations), dNTPs, buffers, capture reagents (e.g., beads, surfaces, coated tubes, columns, etc.), indexing primers, amplification primers (PCR primers) and sequencing primers. In some examples, a kit may include reagents for assessing types of DNA damage such as an error-prone DNA polymerase and/or a high-fidelity DNA polymerase. Additional additives and reagents are contemplated for PCR or ligation reactions in specific conditions (e.g., high GC rich genome/target).

In an example, the kits further comprise reagents, such as DNA error correcting enzymes that repair DNA sequence errors that interfere with polymerase chain reaction (PCR) processes (versus repairing mutations leading to disease). By way of non-limiting example, the enzymes comprise one or more of the following: monofunctional uracil-DNA glycosylase (hSMUG1), Uracil-DNA Glycosylase (UDG), N-glycosylase/AP-lyase NEIL 1 protein (hNEIL1), Formamidopyrimidine DNA glycosylase (FPG), 8-oxoguanine DNA glycosylase (OGG1), human apurinic/apyrimidinic endonuclease (APE 1), endonuclease III (Endo III), endonuclease IV (Endo IV), endonuclease V (Endo V), endonuclease VIII (Endo VIII), T7 endonuclease I (T7 Endo I), T4 pyrimidine dimer glycosylase (T4 PDG), human single-strand-selective human alkyladenine DNA glycosylase (hAAG), etc., among other glycosylases, lyases, endonucleases and exonucleases etc.; and can be utilized to correct DNA damage (e.g., *in vitro* or *in vivo* DNA damage). Some of such DNA repair enzymes, for example, are glycoslyases that remove damaged bases from DNA. For example, UDG removes uracil that results from cytosine deamination (caused by spontaneous hydrolysis of cytosine) and FPG removes 8-oxo-guanine (e.g., most common DNA lesion that results from reactive oxygen species). FPG also has lyase activity that can generate 1 base gap at abasic sites. Such abasic sites will subsequently fail to amplify by PCR, for example, because the polymerase fails copy the template. Accordingly, the use of such DNA damage repair enzymes, and/or others listed here and as known in the art, can effectively remove damaged DNA that does not have a true mutation but might otherwise be undetected as an error.

The kits may further comprise appropriate controls, such as DNA amplification controls, nucleic acid (template) quantification controls, sequencing controls, nucleic acid molecules derived from a similar biological source (e.g., a healthy subject). In some examples, a kit may include a control population of cells. Accordingly, a kit could include suitable reagents (test compounds, nucleic acid, control sequencing library, etc.) for providing controls that would yield expected Duplex Sequencing results that would determine protocol authenticity for samples comprising a rare genetic variant (e.g., nucleic acid molecules comprising disease-associated variants/mutations that can be spiked into or included in the sample preparation steps). In some examples, a kit may include reference sequence information. In some examples, a kit may include sequence information useful for identifying one or more DNA variants in a population of cells or in a cell-free DNA sample. In an example, the kit comprises containers for shipping samples, storage material for stabilizing samples, material for freezing samples, such as cell samples, for analysis to detect DNA variants in a subject sample. In another example, a kit may include nucleic acid contamination control standards (e.g., hybridization capture probes with affinity to genomic regions in an organism that is different than the test or subject organism).

The kit may further comprise one or more other containers comprising materials desirable from a commercial and user standpoint, including PCR and sequencing buffers, diluents, subject sample extraction tools (e.g. syringes, swabs, etc.), and package inserts with instructions for use. In addition, a label can be provided on the container with directions for use, such as those described above; and/or the directions and/or other information can also be included on an insert which is included with the kit; and/or via a website address provided therein. The kit may also comprise laboratory tools such as, for example, sample tubes, plate sealers, microcentrifuge tube openers, labels, magnetic particle separator, foam inserts, ice packs, dry ice packs, insulation, etc.

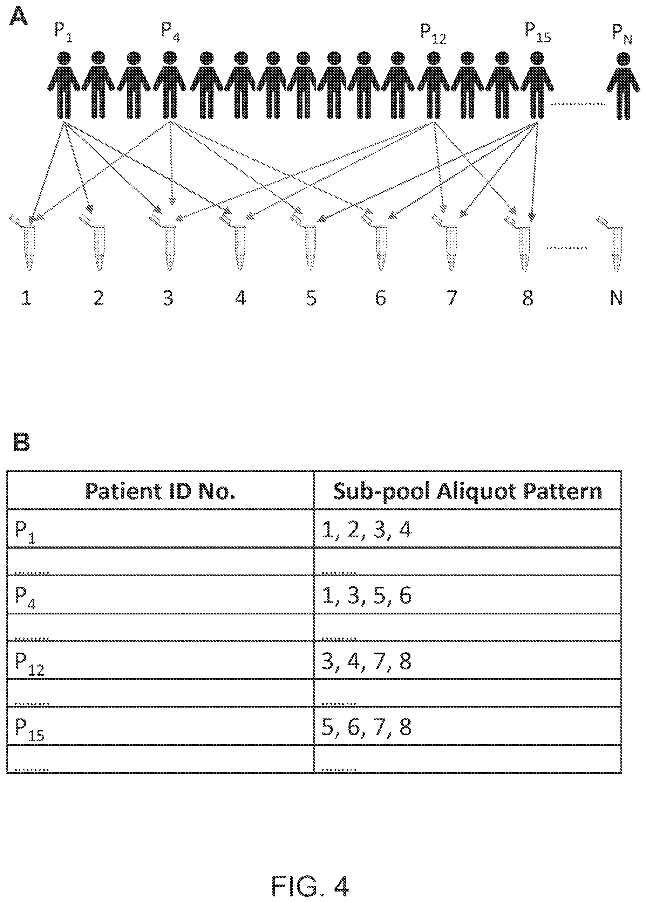

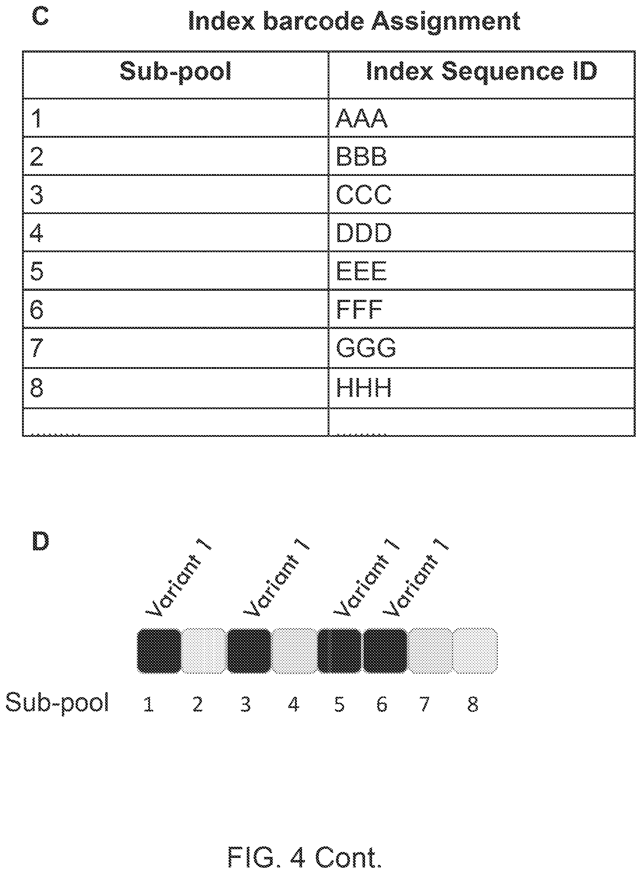

The kits may further comprise a computer program product installable on an electronic computing device (e.g. laptop/desktop computer, tablet, etc.) or accessible via a network (e.g. remote server), wherein the computing device or remote server comprises one or more processors configured to execute instructions to perform operations comprising Duplex Sequencing analysis steps. For example, the processors may be configured to execute instructions for processing raw or unanalyzed sequencing reads to generate Duplex Sequencing data. In additional examples, the computer program product may include a database comprising subject or sample records (e.g., information regarding a particular subject or sample or groups of samples) and empirically-derived information regarding targeted regions of DNA. The computer program product is embodied in a non-transitory computer readable medium that, when executed on a computer, performs steps of the methods disclosed herein (e.g. see FIGS. **4-6**).

The kits may further comprise include instructions and/or access codes/passwords and the like for accessing remote server(s) (including cloud-based servers) for uploading and downloading data (e.g., sequencing data, reports, other data) or software to be installed on a local device. All computational work may reside on the remote server and be accessed by a user/kit user via internet connection, etc.

### Methods of Detection and Treatment

Many rare genetic diseases or disorders are detected only after symptoms have arisen, which can be delayed in life or occur only after irrevocable harm has occurred, but individual genetic testing for the presence of rare genetic alleles is expensive and generally not practical. However, the present technology provides methods of detecting the disease-causing mutations and identifying carriers of genetic alleles such that early intervention is possible. For example, a patient carrying a disease-associated genetic variant and at risk of developing such disease can be treated prophylactically and/or therapeutically to prevent or delay an onset of disease, reduce a severity of disease, and/or reduce a number of symptoms associated with a disease. In other embodiments, subjects that are at higher risk of being carriers of disease-associated variants (e.g., a member of at-risk ethnic group, family history of the disease) can screened, as well as be provided reproductive counseling, if appropriate.

In some embodiments, when a subject is carrier of disease-associated variant allele(s), then the subject is at a significantly increased risk for the onset of the genotoxic disease or disorder (unless the subject is a heterozygous carrier of a variant allele associated with a recessive trait disease). The subject is then treated prophylactically with agents or other therapies suitable to treat the disease or disorder. Additionally, or alternatively, the subject undergoes sequentially timed diagnostic testing (e.g. blood tests, further genotyping, etc.)) and/or imaging (e.g. CAT, MRI, PET, ultrasound, serum biomarker testing, etc.) and/or suitable medical follow-up examinations to detect whether the subject has developed an early stage of the disease or disorder. By way of non-limiting example: for Wilson Disease (e.g., a mutation in the *ATP7B gene*, a P-type ATPase that plays a role in copper transport), the subject would likely be ordered to undergo blood tests, urine tests, a liver ultrasound, and liver biopsy if needed to assess the physical status of the patient. Wilson Disease is treatable and presymptomatic homozygous patients should be treated preventatively. For example, confirmed patients can be put on a typical schedule with heavy metal toxicity medication prior to organ damage.

For many genetic diseases and disorders, methods of providing prophylactic treatments (i.e. prevent or reduce the risk of onset), and/or to reduce the severity of the disorder, comprise treatment protocols well known to the skilled clinician, and would be tailored to the disease or disorder.

### IV. Experimental Examples

The following section provides some limiting examples of methods for genotyping large numbers of samples via pooling using Duplex Sequencing and associated reagents.

### Example 1

A method for pooling patient DNA samples and efficiently genotyping the samples to identify a patient carrying a genetic variant is described in this Example 1. FIG. **4****,** panels **A-D** show the pooling schematic (panel **A**), a look-up table generated from the pooling scheme (panel B), a look-up table generated from a sub-pool indexing scheme (panel **C)**, and identification of sub-pools containing a variant allele (panel **D**) that can be used with look-up tables (panels **B** and **C**) to identify a patient carrying the identified genetic variant in accordance with an embodiment of the present technology.

Referring to FIG. **4****,** panel **A,** biological samples extracted from each patient in a patient population is aliquoted into a unique or substantially unique combinations of sub-pools (e.g., pooled sample mixtures from a subset of the patients). In the non-limiting example shown in panel **A,** patient samples are aliquoted into each of four sub-pools. For illustrative purposes, the sample distribution pattern is only shown for patients 1, 4, 12 and 15 (P₁, P₄, P₁₂, P₁₅). Additionally, while only 15 numbered patients and 8 numbered sub-pools are illustrated, one of ordinary skill in the art will understand that the number of patient samples can be greater or less that than shown (e.g., illustrated is patient "P_{N"}). Likewise, one of ordinary skill in the art will understand that the number of sub-pools may be greater or less than that illustrated in panel **A** (e.g., illustrated is sub-pool "N"). Once a pooling pattern is established such that each sample is aliquoted into a unique or substantially unique combination of sub-pools, the pattern can be recorded in a look-up table, such as shown in panel **B.** The combination of sub-pools serves as a unique sample identifier in this embodiment. For example, the unique sample identifier for patient 1 (P₁) is 1-2-3-4 while the unique sample identifier assigned to patient 4 (P₄) is 1-3-5-6. In the illustrated example, the patient samples are aliquoted into sub-pools with an edit distance of at least 2 between unique sample identifiers (e.g., at least 2 pools are different between each sample sub-pool combination).

Once the sub-pools are populated, sequencing library preparation steps are performed. In one embodiment, indexing sequences are added to adapter-tagged double-stranded DNA molecules such that all sequence reads generated from DNA molecules originating from a particular sub-pool can later be grouped for analysis. For example, and as shown in FIG. **4****,** panel **C,** a look-up table can be generated to correlate that sub-pool identification number (e.g., 1, 2, 3, 4, etc.) with the specific index sequence used. These index sequences are identified, for illustrative purposes only, as AAA, BBB, CCC, etc. One of ordinary skill in the art will understand that a look-up table identifying an index barcode assignment can include an actual nucleotide sequence used, or other code to identify which of a set of index sequences were assigned to a sub-pool.

Following sub-pooled sample mixture indexing, the sub-pools can be combined together and the adapter-tagged DNA molecules in the combined mixture can be sequenced. In one embodiment, Duplex Sequencing can be used to provide highly accurate DNA sequence reads so that variant alleles can be identified. Once sequence reads are error-corrected, they can be grouped according to an original sub-pool assignment by recognition of the index sequence (e.g., AAA, BBB, CCC, etc.) using look-up table (panel **C**).

As illustrated in FIG. **4****,** and in this non-limiting example, a variant allele ("Variant 1") was identified in sub-pools 1, 3, 5, 6. Referring to look-up table in panel **A,** patient 4 (P₄) is identified as the only patient having a DNA sample in the combination of sub-pools 1, 3, 5 and 6. In this example, patient 4 (P₄) is identified as a carrier of Variant 1.

### Example 2

In one example, a drug is known to help patients with loss of function in one of three genes (gene A, gene B, gene C) associated with a disease. The loss of function typically occurs as a result of loss of function variants occurring in both an individual's maternal and paternal alleles for the genes. Additionally, loss of function variants occur at a number sites throughout these genes.

To identify a subpopulation of individuals that would therapeutically benefit from the drug, a biobank of 1,985 patient samples is screened for known loss-of-function mutations in the targeted genomic loci corresponding to genes A, B, and C. Because the target disease variants can fall over many kilobases of the genome, PCR screening for the mutant alleles is not an effective screening tool. NGS can detect variants over all three disease-associated genes, but the cost of individually preparing 1,985 samples for next generation sequencing is very high.

In one example, a protocol is as follows:
Create 40 "sub-pools" to be populated with biobank sample DNA
Aliquot each of the 1,985 samples into a unique combination of 4 sub-pools, such that no two samples share more than 2 sub-pools with each other.

The resulting 40 sub-pools will each comprise an aliquot of an average of approximately 200 samples each.

For an unknown patient in the population having a heterozygous disease allele, such allele will be present at an abundance of 1/400 in any particular sub-pool where that patient's sample has been aliquoted.

If a patient has a compound heterozygous loss of function mutation in a gene, i.e. is heterozygous for two distinct variants in a gene, each of those variants will be present at an abundance of 1/400 in the sub-pools where that patient's sample has been aliquoted.

Conventional NGS has an error rate of approximately 1%, which is too high to allow detection of variants with such low abundance in a sample (e.g., a variant having 1/400 abundance would be indistinguishable from a sequencing error. An error-correction sequencing method is used to detect variants a low abundance, such as 1/400. In one embodiment, the error-correction sequencing method is Duplex Sequencing, In another embodiment, other consensus sequencing methods (e.g., single-strand consensus sequencing) can detect variants at low abundance.

In an example, Duplex Sequence is used to interrogate each of the 40 sub-pools such that 1,985 patient samples are distributed among 40 sub-pools, thereby requiring only 40 sequencing libraries (one for each sub-pooled sample mixture) to be prepared.

Each patient carrying a rare allele can be identified, by identifying the genetic variant(s) in the 4 sub-pools in which his/her DNA was uniquely aliquoted.

The four sub-pools each sample is uniquely aliquoted into serves as a unique sample identifier that is distinguishable from every other unique sample identifier. In a particular example, each sample can have unique sample identifiers that differ by at least two pools. This design is robust to various errors. For example, if a rare variant fails to be detected in one sub-pool, if a sample is accidently aliquoted into an additional sub-pool, if a sample is accidentally aliquoted into one wrong sub-pool, or if DNA molecules comprising the variant fail to provide sequence data in a particular sub-pool, the sample can still be uniquely identifiable.

Results of this example can identify compound heterozygous disease carriers: if an individual carried two distinct disease alleles. Each of these alleles can be associated with the individual based on the unique sample identifier. The disease alleles could fall in either cis or in trans with each other, but a high possibility of the alleles occurring in trans, identifies a compound heterozygote that may be suitable for follow-up confirmatory sequencing.

Additionally, results could identify homozygous disease allele carriers from a large number of samples. For example, if an individual carried two copies of a disease allele, the allele would both be present in the patient's unique sample identifier (e.g., the unique sub-pool set) as well as present at double the allele frequency of a heterozygote variant. Thus, if the sub-pools into which a patient sample has been aliquoted show approximately double the allele frequency expected of a heterozygote variant, the patient can be identified as a putative homozygote carrier and indicated for confirmatory sequencing.

### V. Examples of Systems and Computing Environments for Deconvolution of Complex Mixtures of Genotypes

### Suitable Computing Environments

The following discussion provides a general description of a suitable computing environment in which aspects of the disclosure can be implemented. Although not required, aspects and examples of the disclosure will be described in the general context of computer-executable instructions, such as routines executed by a general-purpose computer, e.g., a server or personal computer. Those skilled in the relevant art will appreciate that the disclosure can be practiced with other computer system configurations, including Internet appliances, hand-held devices, wearable computers, cellular or mobile phones, multi-processor systems, microprocessor-based or programmable consumer electronics, set-top boxes, network PCs, mini-computers, mainframe computers and the like. The disclosure can be embodied in a special purpose computer or data processor that is specifically programmed, configured or constructed to perform one or more of the computer-executable instructions explained in detail below. Indeed, the term "computer", as used generally herein, refers to any of the above devices, as well as any data processor.

The disclosure can also be practiced in distributed computing environments, where tasks or modules are performed by remote processing devices, which are linked through a communications network, such as a Local Area Network ("LAN"), Wide Area Network ("WAN") or the Internet. In a distributed computing environment, program modules or sub-routines may be located in both local and remote memory storage devices. Aspects of the disclosure described below may be stored or distributed on computer-readable media, including magnetic and optically readable and removable computer discs, stored as firmware in chips (e.g., EEPROM chips), as well as distributed electronically over the Internet or over other networks (including wireless networks). Those skilled in the relevant art will recognize that portions of the disclosure may reside on a server computer, while corresponding portions reside on a client computer. Data structures and transmission of data particular to aspects of the disclosure are also encompassed within the scope of the disclosure.

Examples of computers, such as a personal computer or workstation, can comprise one or more processors coupled to one or more user input devices and data storage devices. A computer can also be coupled to at least one output device such as a display device and one or more optional additional output devices (e.g., printer, plotter, speakers, tactile or olfactory output devices, etc.). The computer may be coupled to external computers, such as via an optional network connection, a wireless transceiver, or both.

Various input devices may include a keyboard and/or a pointing device such as a mouse. Other input devices are possible such as a microphone, joystick, pen, touch screen, scanner, digital camera, video camera, and the like. Further input devices can include sequencing machine(s) (e.g., massively parallel sequencer), fluoroscopes, and other laboratory equipment, etc. Suitable data storage devices may include any type of computer-readable media that can store data accessible by the computer, such as magnetic hard and floppy disk drives, optical disk drives, magnetic cassettes, tape drives, flash memory cards, digital video disks (DVDs), Bernoulli cartridges, RAMs, ROMs, smart cards, etc. Indeed, any medium for storing or transmitting computer-readable instructions and data may be employed, including a connection port to or node on a network such as a local area network (LAN), wide area network (WAN) or the Internet.

Aspects of the disclosure may be practiced in a variety of other computing environments. For example, a distributed computing environment with a network interface can include one or more user computers in a system where they may include a browser program module that permits the computer to access and exchange data with the Internet, including web sites within the World Wide Web portion of the Internet. User computers may include other program modules such as an operating system, one or more application programs (e.g., word processing or spread sheet applications), and the like. The computers may be general-purpose devices that can be programmed to run various types of applications, or they may be single-purpose devices optimized or limited to a particular function or class of functions. More importantly, while shown with network browsers, any application program for providing a graphical user interface to users may be employed, as described in detail below; the use of a web browser and web interface are only used as a familiar example here.

At least one server computer, coupled to the Internet or World Wide Web ("Web"), can perform much or all of the functions for receiving, routing and storing of electronic messages, such as web pages, data streams, audio signals, and electronic images that are described herein. While the Internet is shown, a private network, such as an intranet may indeed be preferred in some applications. The network may have a client-server architecture, in which a computer is dedicated to serving other client computers, or it may have other architectures such as a peer-to-peer, in which one or more computers serve simultaneously as servers and clients. A database or databases, coupled to the server computer(s), can store much of the web pages and content exchanged between the user computers. The server computer(s), including the database(s), may employ security measures to inhibit malicious attacks on the system, and to preserve integrity of the messages and data stored therein (e.g., firewall systems, secure socket layers (SSL), password protection schemes, encryption, and the like).

A suitable server computer may include a server engine, a web page management component, a content management component and a database management component, among other features. The server engine performs basic processing and operating system level tasks. The web page management component handles creation and display or routing of web pages. Users may access the server computer by means of a URL associated therewith. The content management component handles most of the functions in the examples described herein. The database management component includes storage and retrieval tasks with respect to the database, queries to the database, read and write functions to the database and storage of data such as video, graphics and audio signals.

Many of the functional units described herein have been labeled as modules, in order to more particularly emphasize their implementation independence. For example, modules may be implemented in software for execution by various types of processors. An identified module of executable code may, for instance, comprise one or more physical or logical blocks of computer instructions which may, for instance, be organized as an object, procedure, or function. The identified blocks of computer instructions need not be physically located together but may comprise disparate instructions stored in different locations which, when joined logically together, comprise the module and achieve the stated purpose for the module.

A module may also be implemented as a hardware circuit comprising custom VLSI circuits or gate arrays, off-the-shelf semiconductors such as logic chips, transistors, or other discrete components. A module may also be implemented in programmable hardware devices such as field programmable gate arrays, programmable array logic, programmable logic devices or the like.

A module of executable code may be a single instruction, or many instructions, and may even be distributed over several different code segments, among different programs, and across several memory devices. Similarly, operational data may be identified and illustrated herein within modules and may be embodied in any suitable form and organized within any suitable type of data structure. The operational data may be collected as a single data set or may be distributed over different locations including over different storage devices, and may exist, at least partially, merely as electronic signals on a system or network.

### System for Efficiently Genotyping Multiple Samples

Also disclosed but not part of the invention is a system (e.g. a networked computer system, a high throughput automated system, etc.) for processing a biological sample comprising a nucleic acid mixture, and transmitting the sequencing data via a wired or wireless network to a server to determine the sample's error-corrected sequence reads (e.g., duplex sequence reads, duplex consensus sequence, etc.), genotype identification, quantification of individual/attributable genotypes, etc.

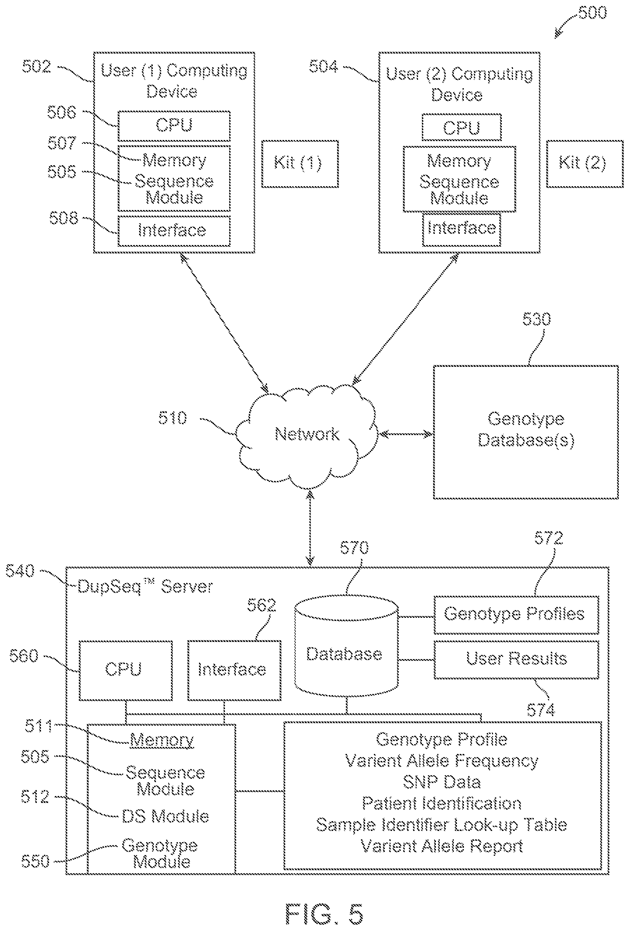

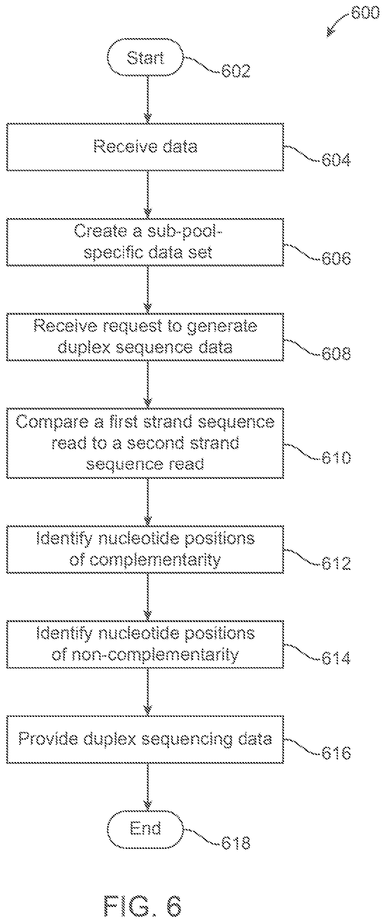

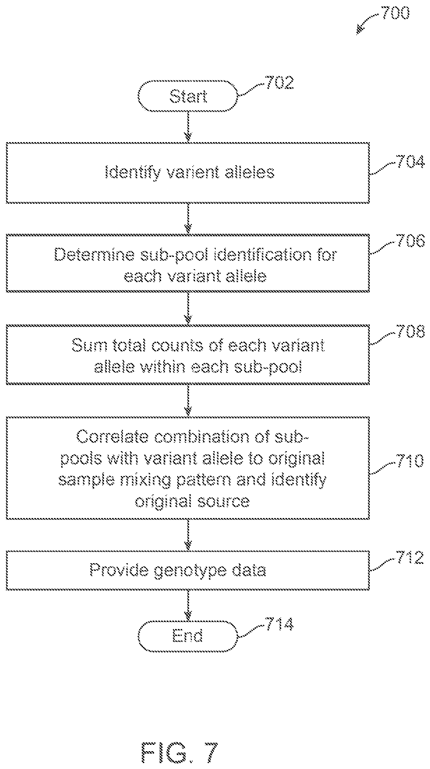

As described in additional detail below, and with respect to the example illustrated in FIG. 5, a computerized system for efficiently genotyping multiple samples comprises: (1) a server (e.g., a remote server, or locally stored server); (2) a plurality of user electronic computing devices able to generate and/or transmit sequencing data; (3) optionally, a database with known genotypes and associated information (optional); and (4) a wired or wireless network for transmitting electronic communications between the electronic computing devices, database, and the server. The server further comprises: (a) a database storing records of genotype profiles (e.g., variant allele profiles), patient identification, empirically-derived disease-associated mutations and associated information; (b) one or more processors communicatively coupled to a memory; and one or more non-transitory computer-readable storage devices or medium comprising instructions for processor(s), wherein said processors are configured to execute said instructions to perform operations comprising one or more of the steps described in FIGS. **6** and **7****.**

In one example, the present technology further comprises, a non-transitory computer-readable storage media comprising instructions that, when executed by one or more processors, performs methods for determining the presence of one or more variant alleles in a sub-pooled sample mixture, the quantification of each identified variant allele in the sub-pooled sample mixture, the identity of a subject/individual from a database who's genetic material is present in the sub-pooled sample mixture, deconvolve sub-pooled sample mixtures of multiple unknown genotypes, and the like. In particular examples, the methods can include one or more of the steps described in FIGS. **6** and **7****.**

Additional aspects of the present technology are directed to computerized methods for determining a presence of one or more variant alleles in a sub-pooled sample mixture, the quantification of each identified variant allele in the sub-pooled sample mixture, the identity of a subject/individual from a database who's genetic material is present in the sub-pooled sample mixture, deconvolve sub-pooled sample mixtures of multiple unknown genotypes, and the like. In particular examples, the methods can include one or more of the steps described in FIGS. **6** and **7****.**

FIG. **5** is a block diagram of a computer system **500** with a computer program product **550** installed thereon and for use with the methods disclosed herein to efficiently genotype multiple biological samples. Although FIG. **5** illustrates various computing system components, it is contemplated that other or different components known to those of ordinary skill in the art, such as those discussed above, can provide a suitable computing environment in which aspects of the disclosure can be implemented. FIG. **6** is a flow diagram illustrating a routine for providing Duplex Sequencing consensus sequence data in accordance with an example of the present technology. FIG. **7** is a flow diagram illustrating various routines for identifying and/or quantifying variant alleles present in sub-pooled sample mixtures and for identifying a subject/individual carrying the variant allele. In accordance with aspects of the present technology, methods described with respect to FIG. **7** can provide sample data including, for example, genotypes present in a sub-pooled sample mixture, the identity of independent biological source represented within the sub-pooled sample mixture that comprise variant alleles, and information derived from comparison of sample data to data sets of known genotypes and VAFs within a population.

As illustrated in FIG. **5****,** the computer system **500** can comprise a plurality of user computing devices **502, 504;** a wired or wireless network **510** and a server ("DupSeq^{™}" server) **540** comprising processors to analyze variant alleles present in multiple samples and to identify individual samples contributing the variant allele(s) (e.g., resolving unique sample identifiers from sub-pooled sample mixtures to identify variant allele contributors from among the multiple samples. In examples, user computing devices **502, 504** can be used to generate and/or transmit sequencing data. In one example, users of computing devices **502, 504** may be those performing other aspects of the present technology such as Duplex Sequencing method steps of biological samples for efficient genotyping of multiple biological sources of genetic material (e.g., for screening such sources for variant disease alleles). In one example, users of computing devices **502, 504** perform certain Duplex Sequencing method steps with a kit (**1, 2**) comprising reagents and/or adapters, in accordance with an example of the present technology, to interrogate biological samples.

As illustrated, each user computing device **502, 504** includes at least one central processing unit **506,** a memory **507** and a user and network interface **508.** In an example, the user devices **502, 504** comprise a desktop, laptop, or a tablet computer.

Although two user computing devices **502, 504** are depicted, it is contemplated that any number of user computing devices may be included or connected to other components of the system **500.** Additionally, computing devices **502, 504** may also be representative of a plurality of devices and software used by User (1) and User (2) to amplify and sequence the samples. For example, a computing device may a sequencing machine (e.g., Illumina HiSeq^{™}, Ion Torrent^{™} PGM, ABI SOLiD^{™} sequencer, PacBio RS, Helicos Heliscope^{™}, etc.), a real-time PCR machine (e.g., ABI 7900, Fluidigm BioMark^{™}, etc.), a microarray instrument, etc.

In addition to the above described components, the system **500** may further comprise a database **530** for storing genotype profiles, patient identification, disease-associated mutations and associated information. For example, the database **530,** which can be accessible by the server **540,** can comprise records or collections of disease-associated mutations, population-based genetic information, and sub-pooling reference charts for resolving patient identification for samples carrying a disease-associated variant allele (e.g., sample pooling pattern information). In a particular example, the database **530** can be a third-party database comprising genotype profiles **532** (e.g., known disease-associated mutations, patient genotypes at one or more genomic loci). For example, various genetic research databases comprising nucleic acid sequences for disease-associated genomic loci can be queried for particular applications. In another example, the database can be a standalone database **530** (private or not private) hosted separately from server **540,** or a database can be hosted on the server **540,** such as database **570,** that comprises empirically derived genotype profiles **572.** In some examples, as the system **500** is used to generate new genotype profiles, the data generated from use of the system **500** and associated methods (e.g., methods described herein and, for example, in FIGS. **6-7**), can be uploaded to the database **530** and/or **570** so additional genotype profiles **532, 572** can be created for future comparison activities.

The server **540** can be configured to receive, compute and analyze sequencing data (e.g., raw sequencing files) and related information from user computing devices **502, 504** via the network **510.** Sample-specific raw sequencing data can be computed locally using a computer program product/module (Sequence Module **505**) installed on devices **502, 504,** or accessible from the server **540** via the network **510,** or using other sequencing software well known in the art. The raw sequence data can then be transmitted via the network **510** to the server **540** and user results **574** can be stored in database **570.** The server **540** also comprises program product/module "DS Module" **512** configured to receive the raw sequencing data from the database **570** and configured to computationally generate error corrected double-stranded sequence reads using, for example, Duplex Sequencing techniques disclosed herein. While DS Module **512** is shown on server **540,** one of ordinary skill in the art would recognize that DS Module **512** can alternatively, be hosted at operated at devices **502, 504** or on another server (not shown).

The server **540** can comprise at least one central processing unit (CPU) **560,** a user and a network interface **562** (or server-dedicated computing device with interface connected to the server), a database **570,** such as described above, with a plurality of computer files/records to store genotype profiles of known and unknown biological sources **572,** and files/records to store results (e.g., raw sequencing data, Duplex Sequencing data, variant allele analysis, individual sample identification, etc.) for tested samples **574.** Server **540** further comprises a computer memory **511** having stored thereon the Genotype Computer Program Product (Genotype Module) **550,** in accordance with aspects of the present technology.

Computer program product/module **550** is embodied in a non-transitory computer readable medium that, when executed on a computer (e.g. server **540**), performs steps of the methods disclosed herein for detecting genomic variants (e.g., rare variants) and efficiently identifying an individual source of the genomic variants from among multiple sources, resolving sub-pooled sample mixtures into individual genotypes, and/or quantifying the VAF among the sub-pooled sample mixture. Another aspect of the present disclosure comprises the computer program product/module **550** comprising a non-transitory computer-usable medium having computer-readable program codes or instructions embodied thereon for enabling a processor to carry out genotype analysis (e.g. compute variant alleles, quantify identified variant alleles, resolve mixtures into contributing biological sources, genotype comparison reports, etc.). These computer program instructions may be loaded onto a computer or other programmable apparatus to produce a machine, such that the instructions which execute on the computer or other programmable apparatus create means for implementing the functions or steps described herein. These computer program instructions may also be stored in a computer-readable memory or medium that can direct a computer or other programmable apparatus to function in a particular manner, such that the instructions stored in the computer-readable memory or medium produce an article of manufacture including instruction means which implement the analysis. The computer program instructions may also be loaded onto a computer or other programmable apparatus to cause a series of operational steps to be performed on the computer or other programmable apparatus to produce a computer implemented process such that the instructions which execute on the computer or other programmable apparatus provide steps for implementing the functions or steps described above.

Furthermore, computer program product/module **550** may be implemented in any suitable language and/or browsers. For example, it may be implemented with Python, Java, Scala, C language and preferably using object-oriented high-level programming languages such as Visual Basic, Small Talk, C++, and the like. The application can be written to suit environments such as the Microsoft Windows^{™} environment including Windows^{™} 98, Windows^{™} 2000, Windows^{™} NT, and the like. In addition, the application can also be written for the Macintosh^{™}, SUN^{™}, UNIX or LINUX environment. In addition, the functional steps can also be implemented using a universal or platform-independent programming language. Examples of such multi-platform programming languages include, but are not limited to, hypertext markup language (HTML), JAVA^{™}, JavaScript^{™}, Flash programming language, common gateway interface/structured query language (CGI/SQL), practical extraction report language (PERL), AppleScript^{™} and other system script languages, programming language/structured query language (PL/SQL), and the like. Java^{™}- or JavaScript^{™}-enabled browsers such as HotJava^{™}, Microsoft^{™} Explorer^{™}, or Firefox^{™} can be used. When active content web pages are used, they may include Java^{™} applets or ActiveX^{™} controls or other active content technologies.

The system invokes a number of routines. While some of the routines are described herein, one skilled in the art is capable of identifying other routines the system could perform. Moreover, the routines described herein can be altered in various ways. As examples, the order of illustrated logic may be rearranged, substeps may be performed in parallel, illustrated logic may be omitted, other logic may be included, etc.

FIG. **6** is a flow diagram illustrating routine **600** for providing Duplex Sequencing Data for double-stranded nucleic acid molecules in a sample (e.g., a sample from a sub-pooled sample mixture). The routine **600** can be invoked by a computing device, such as a client computer or a server computer coupled to a computer network. In one example the computing device includes sequence data generator and/or a sequence module. As an example, the computing device may invoke the routine **600** after an operator engages a user interface in communication with the computing device.

The routine **600** begins at block **602** and the sequence module receives raw sequence data from a user computing device (block **604**) and creates a sub-pool-specific data set comprising a plurality of raw sequence reads derived from a plurality of nucleic acid molecules in the sub-pool (block **606**). In some examples, the server can store the sub-pool-specific data set in a database for later processing. Next, the DS module receives a request for generating Duplex Consensus Sequencing data from the raw sequence data in the sub-pool-specific data set (block **608**). The DS module groups sequence reads from families representing an original double-stranded nucleic acid molecule (e.g., based on SMI sequences) and compares representative sequences from individual strands to each other (block **610**). In one example, the representative sequences can be one or more than one sequence read from each original nucleic acid molecule. In another example, the representative sequences can be single-strand consensus sequences (SSCSs) generated from alignment and error-correction within representative strands. In such examples, a SSCS from a first strand can be compared to a SSCS from a second strand.

At block **612,** the DS module identifies nucleotide positions of complementarity between the compared representative strands. For example, the DS module identifies nucleotide positions along the compared (e.g., aligned) sequence reads where the nucleotide base calls are in agreement. Additionally, the DS module identifies positions of non-complementarity between the compared representative strands (block **614**). Accordingly, the DS module can identify nucleotide positions along the compared (e.g., aligned) sequence reads where the nucleotide base calls are in disagreement.

Next, the DS module can provide Duplex Sequencing Data for double-stranded nucleic acid molecules in a sub-pooled sample mixture (block **616**). Such data can be in the form of duplex consensus sequences for each of the processed sequence reads. Duplex consensus sequences can include, in one example, only nucleotide positions where the representative sequences from each strand of an original nucleic acid molecule are in agreement. Accordingly, in one example, positions of disagreement can be eliminated or otherwise discounted such that the duplex consensus sequence is a high accuracy sequence read that has been error-corrected. In another example, Duplex Sequencing Data can include reporting information on nucleotide positions of disagreement in order that such positions can be further analyzed (e.g., in instances where DNA damage can be assessed). The routine **600** may then continue at block **618,** where it ends.

FIG. **7** is a flow diagram illustrating a routine **700** for detecting, identifying and quantifying variant alleles present in nucleic acid mixtures to identify an original contributing source of the variant allele(s). The routine can be invoked by the computing device of FIG. **5****.** The routine **700** begins at block **702** and the genotype module analyzes the Duplex Sequencing Data from FIG. **6** (e.g., following block **616**) to identify variant allele(s) present within individual DNA molecules (block **704**), determine a sub-pool identification for each variant allele present (block **706**) and sums the total counts of each variant allele within each sub-pool (block **708**). Next, the genotype module correlates the combination of sub-pools comprising the variant allele(s) to an original sample mixing pattern to identify an original source of the variant allele among a population of sources (block **710**). As such, a variant allele analysis of sub-pooled sample mixtures can provide information regarding the original biological source contributing to the nucleic acid mixture.

Next, the genotype module can provide genotype data (block **712**) that can be stored in the sub-pool-specific or a sample-specific data set in the database. The routine **700** may then continue at block **714,** where it ends.

### VII. Conclusion

While steps are presented in a given order, alternative embodiments may perform steps in a different order in accordance with the claims. The various embodiments described herein may also be combined to provide further embodiments in accordance with the claims.

From the foregoing, it will be appreciated that specific embodiments of the technology have been described herein for purposes of illustration, but well-known structures and functions have not been shown or described in detail to avoid unnecessarily obscuring the description of the embodiments of the technology. Where the context permits, singular or plural terms may also include the plural or singular term, respectively.

Moreover, unless the word "or" is expressly limited to mean only a single item exclusive from the other items in reference to a list of two or more items, then the use of "or" in such a list is to be interpreted as including (a) any single item in the list, (b) all of the items in the list, or (c) any combination of the items in the list. Additionally, the term "comprising" is used throughout to mean including at least the recited feature(s) such that any greater number of the same feature and/or additional types of other features are not precluded. It will also be appreciated that specific embodiments have been described herein for purposes of illustration, but that various modifications may be made without deviating from the technology in accordance with the claims. Further, while advantages associated with certain embodiments of the technology have been described in the context of those embodiments, other embodiments may also exhibit such advantages, and not all embodiments need necessarily exhibit such advantages to fall within the scope of the technology and in accordance with the claims. Accordingly, the disclosure and associated technology can encompass other embodiments not expressly shown or described herein but in accordance with the claims.

## Claims

1. A method for genotyping a plurality of biological samples via pooling, comprising:
pooling the plurality of biological samples, which each comprise a plurality of target
double-stranded DNA molecules, into a unique combination of sub-pools, generating an error-corrected sequence read for each of a plurality of the target double-
stranded DNA molecules in the sub-pools;
identifying a presence of one or more variant alleles from the error-corrected sequence reads; and
determining the original biological sample containing the variant allele(s) by identifying the unique combination of sub-pools containing the variant allele(s), wherein the error corrected sequence read is generated by:
(i) single-strand consensus sequencing;
(ii) a combination of single-strand and duplex consensus sequencing, or;
(iii) a method comprising:
ligating adapter molecules to the plurality of target double-stranded DNA molecules to generate a plurality of adapter-DNA molecules;
for each of a plurality of adapter-DNA molecules, generating a set of copies of an original first strand of the adapter-DNA molecule and a set of copies of an original second strand of the adapter-DNA molecule;
sequencing one or more copies of the original first and second strands to provide a first strand sequence and a second strand sequence; and
comparing the first strand sequence and the second strand sequence to identify one or more correspondences between the first and second strand sequences.

2. The method of claim 1, wherein the method is for screening biological sources for a genetic variant, and the step of pooling comprises:
aliquoting the plurality of biological samples, which are derived from the biological sources, into the unique combination of sub-pools.

3. The method of any one of claims 1-2, wherein generating an error-corrected sequence read for each of a plurality of the target double-stranded DNA molecules in the sub-pools further comprises selectively enriching one or more targeted genomic regions prior to sequencing.

4. The method of claim 3, wherein the one or more targeted genomic regions comprise genes known to harbor disease-causing mutations, optionally wherein a disease-causing mutation is or includes a loss of function mutation, a gain of function mutation, or a dominant negative mutation.

5. The method of claims 3 or 4, wherein the one or more targeted genomic regions comprise genetic loci known to be associated with a disease or disorder, optionally wherein the disease or disorder:-
(a) is a rare genetic disorder;
(b) is a single-gene disorder or a complex disorder involving mutations in two or more genes;
(c) is associated with an autosomal recessive mutation;
(d) is associated with an autosomal dominant mutation; or
(e) comprises Phenylketonuria (PKU), Cystic fibrosis, Sickle-cell anemia, Albinism, Huntington's disease, Myotonic dystrophy type 1, Hypercholesterolemia, Neurofibromatosis, Polycystic kidney disease 1 and 2, Hemophilia A, Muscular dystrophy (Duchenne type), Hypophosphatemic rickets, Rett's syndrome, Tay-Sachs disease, Wilson disease, and/or Spermatogenic failure.

6. The method of any one of claims 1-5, wherein identifying a presence of one or more variant allele(s) from the error-corrected sequence reads comprises comparing the error-corrected sequence reads to a reference genome DNA sequence.

7. The method of any one of claims 1-6, further comprising determining a frequency of the one or more variants among the plurality of target double-stranded DNA molecules in each sub-pool, optionally further determining if a biological source donor of the biological sample comprising the variant allele(s) is heterozygous or homozygous for the variant allele.

8. The method of any one of claims 4-7, wherein the one or more targeted genomic regions comprise a cancer driver, a proto-oncogene, a tumor suppressor gene and/or an oncogene, optionally wherein the cancer driver comprises *ABL, ACC, BCR, BLCA, BRCA, CESC, CHOL, COAD, DLBC, DNMT3A, EGFR, ESCA, GBM, HNSC, KICH, KIRC, KIRP, LAML, LGG, LIHC, LUAD, LUSC, MESO, OV, PAAD, PCPG, PI3K, PIK3CA, PRAD, PTEN, RAS, READ, SARC, SKCM, STAD, TGCT, THCA, THYM, TP53, UCEC, UCS,* and/or *UVM.*

9. The method of claim 3, wherein the one or more targeted genomic regions comprise (a) a gene associated with a rare autoimmune, metabolic or neurological genetic disorder or disease; or (b) a genetic locus associated with rare genetic disorders of obesity, optionally wherein the rare genetic disorders of obesity are or include Proopiomelanocortin (POMC) Deficiency Obesity, Alström syndrome, Leptin Receptor (LEPR) Deficiency Obesity, Prader-Willi syndrome (PWS), Bardet-Biedl syndrome (BBS), and high-impact Heterozygous Obesity.

10. The method of any one of claims 1-9, wherein the target double-stranded DNA molecules are extracted from a blood draw taken from a human.

11. The method of claim 1, comprising:
aliquoting the plurality of biological samples into a plurality of sub-pools, such that no two biological samples are aliquoted into the same combination of sub-pools;
generating duplex sequencing data from raw sequencing data, wherein the raw sequencing data is generated from the plurality of sub-pooled biological samples comprising the target double-stranded DNA molecules, and wherein the target double-stranded DNA molecules comprise one or more genetic variants; and
identifying a donor source of the one or more genetic variants present in the sub-pooled biological samples by identifying the unique combination of sub-pools containing the one or more genetic variants.

12. The method of claim 11, wherein for each sub-pool, the method further comprises:
(a) preparing a sequencing library from the aliquoted biological samples, wherein preparing the sequence library comprises ligating asymmetric adapter molecules to the plurality of target double-stranded DNA molecules in the sub-pool to generate a plurality of adapter-DNA molecules;
(b) sequencing first and second strands of the adapter-DNA molecules to provide a first strand sequence read and a second strand sequence read for each adapter-DNA molecule of the plurality of adapter-DNA molecules; and
(c) for each adapter-DNA molecule, comparing the first strand sequence read and the second strand sequence read to identify one or more correspondences between the first and second strand sequence reads to provide the error-corrected sequence reads for each of a plurality of the target double-stranded DNA molecules in the sub-pools, optionally wherein prior to sequencing in step (b), the method further comprises combining the adapter-DNA molecules from the sub-pools; and optionally wherein
the adapter molecules have an indexing sequence;
each sub-pool is tagged using a unique indexing sequence; and
wherein identifying the unique combination of sub-pools comprises identifying the indexing sequence associated with each genetic variant of the one or more genetic variants, and optionally further comprising cross-referencing the indexing sequence associated with each genetic variant to the combination of sub-pools each biological sample is aliquoted to identify the donor source.

13. The method of any one of claims 11-12, wherein generating the error-corrected sequence read for each of a plurality of the target double-stranded DNA molecules in the sub-pools further comprises selectively enriching one or more targeted genomic loci prior to sequencing to provide a plurality of enriched adapter-DNA molecules.

14. The method of any one of claims 1-13, wherein the number of sub-pools is or comprises (a) 10, 11, 12, 13, 14, 15, 16, 17, 18, 19, 20, 21, 22, 23, 24, 25, 26, 27, 28, 29, 30, 31, 32, 33, , 35, 36, 37, 38, 39, 40, 42, 45, 47, 50, 52, 55, 57, 60, 62, 65, 67, or 70 sub-pools or (b) between about 15 and about 40 sub-pools, between about 30 and about 50 sub-pools, between about 35 and about 55 sub-pools, between about 40 and about 60 sub-pools, or over 60 sub-pools.

## Patentansprüche

1. Verfahren zur Genotypisierung einer Vielzahl von biologischen Proben über Pooling, umfassend:
Poolen der Vielzahl von biologischen Proben, die jeweils eine Vielzahl von doppelsträngigen DNA-Zielmolekülen umfassen, in eine einzigartige Kombination von Teilpools,
Erzeugen einer fehlerkorrigierten Sequenzlesung für jedes aus einer Vielzahl der doppelsträngigen DNA-Zielmoleküle in den Teilpools;
Identifizieren eines Vorhandenseins von einem oder mehreren varianten Allelen aus den fehlerkorrigierten Sequenzlesungen; und
Bestimmen der ursprünglichen biologischen Probe, die das/die variante(n) Allel(e) enthält, durch Identifizieren der einzigartigen Kombination von Teilpools, die das/die variante(n) Allel(e) enthält,
wobei die fehlerkorrigierte Sequenzlesung durch Folgendes erzeugt wird:
(i) Einzelstrang-Konsens-Sequenzierung;
(ii) eine Kombination aus Einzelstrang- und Duplex-Konsens-Sequenzierung, oder;
(iii) ein Verfahren, umfassend:
Ligieren von Adaptermolekülen an die Vielzahl von doppelsträngigen DNA-Zielmolekülen, um eine Vielzahl von Adapter-DNA-Molekülen zu erzeugen;
für jedes aus einer Vielzahl von Adapter-DNA-Molekülen, Erzeugen eines Satzes von Kopien eines ursprünglichen ersten Strangs des Adapter-DNA-Moleküls und eines Satzes von Kopien eines ursprünglichen zweiten Strangs des Adapter-DNA-Moleküls;
Sequenzieren von einer oder mehreren Kopien des ursprünglichen ersten und zweiten Strangs, um eine erste Strangsequenz und eine zweite Strangsequenz bereitzustellen; und
Vergleichen der ersten Strangsequenz und der zweiten Strangsequenz, um eine oder mehrere Entsprechungen zwischen der ersten und der zweiten Strangsequenz zu identifizieren.

2. Verfahren nach Anspruch 1, wobei das Verfahren zum Screenen von biologischen Quellen auf eine genetische Variante ist und der Schritt des Poolens Folgendes umfasst:
Aliquotieren der Vielzahl von biologischen Proben, die aus den biologischen Quellen stammen, in die einzigartige Kombination von Teilpools.

3. Verfahren nach einem der Ansprüche 1-2, wobei das Erzeugen einer fehlerkorrigierten Sequenzlesung für jedes aus einer Vielzahl der doppelsträngigen DNA-Zielmoleküle in den Teilpools ferner selektives Anreichern von einer oder mehreren targetierten genomischen Regionen vor dem Sequenzieren umfasst.

4. Verfahren nach Anspruch 3, wobei die eine oder mehreren targetierten genomischen Regionen Gene umfassen, von denen bekannt ist, dass sie krankheitsverursachende Mutationen beherbergen, wobei optional eine krankheitsverursachende Mutation eine Funktionsverlustmutation, eine Funktionsgewinnmutation oder eine dominant negative Mutation ist oder beinhaltet.

5. Verfahren nach Anspruch 3 oder 4, wobei die eine oder mehreren targetierten genomischen Regionen genetische Loci umfassen, von denen bekannt ist, dass sie mit einer Krankheit oder Störung assoziiert sind, wobei optional die Krankheit oder Störung:-
(a) eine seltene genetische Störung ist;
(b) eine Einzelgenstörung oder eine komplexe Störung ist, die Mutationen in zwei oder mehr Genen involviert;
(c) mit einer autosomal rezessiven Mutation assoziiert ist;
(d) mit einer autosomal dominanten Mutation assoziiert ist; oder
(e) Phenylketonurie (PKU), zystische Fibrose, Sichelzellenanämie, Albinismus, Huntington-Krankheit, myotonische Dystrophie Typ 1, Hypercholesterinämie, Neurofibromatose, polyzystische Nierenkrankheit 1 und 2, Hämophilie A, Muskeldystrophie (Duchenne-Typ), hypophosphatämische Rachitis, Rett-Syndrom, Tay-Sachs-Krankheit, Wilson-Krankheit und/oder spermatogenes Versagen umfasst.

6. Verfahren nach einem der Ansprüche 1-5, wobei das Identifizieren eines Vorhandenseins von einem oder mehreren varianten Allel(en) aus den fehlerkorrigierten Sequenzlesungen Vergleichen der fehlerkorrigierten Sequenzlesungen mit einer Referenzgenom-DNA-Sequenz umfasst.

7. Verfahren nach einem der Ansprüche 1-6, ferner umfassend Bestimmen einer Häufigkeit der einen oder mehreren Varianten aus der Vielzahl von doppelsträngigen DNA-Zielmolekülen in jedem Teilpool, optional ferner Bestimmen, ob ein Spender aus biologischer Quelle der biologischen Probe, der das/die variante(n) Allel(e) umfasst, heterozygot oder homozygot für das variante Allel ist.

8. Verfahren nach einem der Ansprüche 4-7, wobei die eine oder mehreren targetierten genomischen Regionen einen Krebstreiber, ein Proto-Onkogen, ein Tumorsuppressorgen und/oder ein Onkogen umfassen, wobei optional der Krebstreiber *ABL, ACC, BCR, BLCA, BRCA, CESC, CHOL, COAD, DLBC, DNMT3A, EGFR, ESCA, GBM, HNSC, KICH, KIRC, KIRP, LAML, LGG, LIHC, LUAD, LUSC, MESO, OV, PAAD, PCPG, PI3K, PIK3CA, PRAD, PTEN, RAS, READ, SARC, SKCM, STAD, TGCT, THCA, THYM, TP53, UCEC, UCS* und/oder *UVM* umfasst.

9. Verfahren nach Anspruch 3, wobei die eine oder mehreren targetierten genomischen Regionen (a) ein Gen assoziiert mit einer seltenen autoimmunen, metabolischen oder neurologischen genetischen Störung oder Krankheit; oder (b) einen genetischen Locus assoziiert mit seltenen genetischen Störungen von Fettleibigkeit umfassen, wobei optional die seltenen genetischen Störungen von Fettleibigkeit Proopiomelanocortin(POMC-)Mangel-Adipositas, Alström-Syndrom, Leptin-Rezeptor(LEPR-)Mangel-Adipositas, Prader-Willi-Syndrom (PWS), Bardet-Biedl-Syndrom (BBS) und hochwirksame heterozygote Adipositas sind oder beinhalten.

10. Verfahren nach einem der Ansprüche 1-9, wobei die doppelsträngigen DNA-Zielmoleküle aus einer Blutentnahme von einem Menschen extrahiert sind.

11. Verfahren nach Anspruch 1, umfassend:
Aliquotieren der Vielzahl von biologischen Proben in eine Vielzahl von Teilpools, sodass keine zwei biologischen Proben in die gleiche Kombination von Teilpools aliquotiert sind;
Erzeugen von Duplex-Sequenzierungsdaten aus Rohsequenzierungsdaten, wobei die Rohsequenzierungsdaten aus der Vielzahl von teilgepoolten biologischen Proben erzeugt werden, welche die doppelsträngigen DNA-Zielmoleküle umfassen, und wobei die doppelsträngigen DNA-Zielmoleküle eine oder mehrere genetische Varianten umfassen; und
Identifizieren einer Spenderquelle der einen oder mehreren genetischen Varianten, die in den teilgepoolten biologischen Proben vorhanden sind, durch Identifizieren der einzigartigen Kombination von Teilpools, welche die eine oder mehreren genetischen Varianten enthalten.

12. Verfahren nach Anspruch 11, wobei für jeden Teilpool das Verfahren ferner Folgendes umfasst:
(a) Herstellen einer Sequenzierungsbibliothek aus den aliquotierten biologischen Proben, wobei das Herstellen der Sequenzbibliothek Ligieren von asymmetrischen Adaptermolekülen an die Vielzahl von doppelsträngigen DNA-Zielmolekülen in dem Teilpool umfasst, um eine Vielzahl von Adapter-DNA-Molekülen zu erzeugen;
(b) Sequenzieren von ersten und zweiten Strängen der Adapter-DNA-Moleküle, um eine erste Strangsequenzlesung und eine zweite Strangsequenzlesung für jedes Adapter-DNA-Molekül aus der Vielzahl von Adapter-DNA-Molekülen bereitzustellen; und
(c) für jedes Adapter-DNA-Molekül Vergleichen der ersten Strangsequenzlesung und der zweiten Strangsequenzlesung, um eine oder mehrere Entsprechungen zwischen der ersten und der zweiten Strangsequenzlesung zu identifizieren, um die fehlerkorrigierten Sequenzlesungen für jedes aus einer Vielzahl der doppelsträngigen DNA-Zielmoleküle in den Teilpools bereitzustellen, wobei optional vor dem Sequenzieren in Schritt (b) das Verfahren ferner Kombinieren der Adapter-DNA-Moleküle aus den Teilpools umfasst; und wobei optional
die Adaptermoleküle eine Indexierungssequenz aufweisen;
jeder Teilpool unter Verwendung einer einzigartigen Indexierungssequenz markiert ist; und
wobei das Identifizieren der einzigartigen Kombination von Teilpools Identifizieren der Indexierungssequenz assoziiert mit jeder genetischen Variante der einen oder mehreren genetischen Varianten umfasst, und optional ferner umfassend Querverweisen der Indexierungssequenz assoziiert mit jeder genetischen Variante auf die Kombination von Teilpools, wobei jede biologische Probe aliquotiert ist, um die Spenderquelle zu identifizieren.

13. Verfahren nach einem der Ansprüche 11-12, wobei das Erzeugen der fehlerkorrigierten Sequenzlesung für jedes aus einer Vielzahl der doppelsträngigen DNA-Zielmoleküle in den Teilpools ferner selektives Anreichern von einem oder mehreren targetierten genomischen Loci vor dem Sequenzieren umfasst, um eine Vielzahl von angereicherten Adapter-DNA-Molekülen bereitzustellen.

14. Verfahren nach einem der Ansprüche 1-13, wobei die Anzahl an Teilpools (a) 10, 11, 12, 13, 14, 15, 16, 17, 18, 19, 20, 21, 22, 23, 24, 25, 26, 27, 28, 29, 30, 31, 32, 33,, 35, 36, 37, 38, 39, 40, 42, 45, 47, 50, 52, 55, 57, 60, 62, 65, 67 oder 70 Teilpools oder (b) zwischen etwa 15 und etwa 40 Teilpools, zwischen etwa 30 und etwa 50 Teilpools, zwischen etwa 35 und etwa 55 Teilpools, zwischen etwa 40 und etwa 60 Teilpools oder über 60 Teilpools ist oder umfasst.

## Revendications

1. Procédé pour le génotypage d'une pluralité d'échantillons biologiques par regroupement, comprenant :
le regroupement de la pluralité d'échantillons biologiques, qui comprennent chacun une pluralité de molécules d'ADN double brin cibles, en une combinaison unique de sous-groupes,
la génération d'une séquence avec correction d'erreurs lue pour chacune d'une pluralité des molécules d'ADN double brin cibles dans les sous-groupes ;
l'identification d'une présence d'un ou plusieurs allèles variants à partir des lectures de séquences avec correction d'erreurs ; et
la détermination de l'échantillon biologique d'origine contenant le(s) allèle(s) variant(s) en identifiant la combinaison unique de sous-groupes contenant le(s) allèle(s) variant(s), ladite séquence avec correction d'erreurs lue étant générée par :
(i) le séquençage consensus simple brin ;
(ii) une combinaison de séquençage consensus simple brin et duplex, ou ;
(iii) un procédé comprenant :
la ligature de molécules d'adaptateur à la pluralité de molécules d'ADN double brin cibles afin de générer une pluralité de molécules ADN-adaptateur ;
pour chacune d'une pluralité de molécules ADN-adaptateur, la génération d'un ensemble de copies d'un premier brin d'origine de la molécule ADN-adaptateur et d'un ensemble de copies d'un second brin d'origine de la molécule ADN-adaptateur ;
le séquençage d'une ou de plusieurs copies des premier et second brins d'origine afin de fournir une séquence de premier brin et une séquence de second brin ; et
la comparaison de la séquence de premier brin et de la séquence de second brin afin d'identifier une ou plusieurs correspondances entre les séquences de premier et second brins.

2. Procédé de la revendication 1, dans lequel le procédé est destiné au criblage de sources biologiques pour un variant génétique, et l'étape de regroupement comprend :
l'aliquotage de la pluralité d'échantillons biologiques, qui sont dérivés des sources biologiques, dans la combinaison unique de sous-groupes.

3. Procédé de l'une quelconque des revendications 1 à 2, dans lequel la génération d'une séquence avec correction d'erreurs lue pour chacune d'une pluralité de molécules d'ADN double brin cibles dans les sous-groupes comprend en outre l'enrichissement sélectif d'une ou plusieurs régions génomiques ciblées avant le séquençage.

4. Procédé de la revendication 3, dans lequel la ou les régions génomiques ciblées comprennent des gènes connus pour abriter des mutations pathogènes, éventuellement dans lequel une mutation pathogène est ou comprend une mutation de perte de fonction, une mutation de gain de fonction ou une mutation négative dominante.

5. Procédé des revendications 3 ou 4, dans lequel la ou les régions génomiques ciblées comprennent des loci génétiques connus pour être associés à une maladie ou un trouble, éventuellement ladite maladie ou ledit trouble :
(a) est un trouble génétique rare ;
(b) est un trouble monogénique ou un trouble complexe impliquant des mutations dans deux gènes ou plus ;
(c) est associé(e) à une mutation autosomique récessive ;
(d) est associé(e) à une mutation autosomique dominante ; ou
(e) comprend la phénylcétonurie (PKU), la fibrose kystique, la drépanocytose, l'albinisme, la maladie de Huntington, la dystrophie myotonique de type 1, l'hypercholestérolémie, la neurofibromatose, la polykystose rénale 1 et 2, l'hémophilie A, la dystrophie musculaire (de type Duchenne), le rachitisme hypophosphatémique, le syndrome de Rett, la maladie de Tay-Sachs, la maladie de Wilson et/ou l'insuffisance spermatogénique.

6. Procédé de l'une quelconque des revendications 1 à 5, dans lequel l'identification d'une présence d'un ou de plusieurs allèles variants à partir des lectures de séquences avec correction d'erreurs comprend la comparaison des lectures de séquences avec correction d'erreurs à une séquence d'ADN de génome de référence.

7. Procédé de l'une quelconque des revendications 1 à 6, comprenant en outre la détermination d'une fréquence du ou des variants parmi la pluralité de molécules d'ADN double brin cibles dans chaque sous-groupe, éventuellement en outre la détermination si un donneur source biologique de l'échantillon biologique comprenant le ou les allèles variants est hétérozygote ou homozygote pour l'allèle variant.

8. Procédé de l'une quelconque des revendications 4 à 7, dans lequel la ou les régions génomiques ciblées comprennent un conducteur du cancer, un proto-oncogène, un gène suppresseur de tumeur et/ou un oncogène, éventuellement dans lequel le conducteur du cancer comprend *ABL, ACC, BCR, BLCA, BRCA, CESC, CHOL, COAD, DLBC, DNMT3A, EGFR, ESCA, GBM, HNSC, KICH, KIRC, KIRP, LAML, LGG, LIHC, LUAD, LUSC, MESO, OV, PAAD, PCPG, PI3K, PIK3CA, PRAD, PTEN, RAS, READ, SARC, SKCM, STAD, TGCT, THCA, THYM, TP53, UCEC, UCS* et/ou *UVM.*

9. Procédé de la revendication 3, dans lequel la ou les régions génomiques ciblées comprennent (a) un gène associé à un trouble ou une maladie génétique rare auto-immun(e), métabolique ou neurologique ; ou (b) un locus génétique associé à des troubles génétiques rares de l'obésité, éventuellement dans lequel les troubles génétiques rares de l'obésité sont ou comprennent l'obésité par déficit en proopiomélanocortine (POMC), le syndrome d'Alström, l'obésité par déficit en récepteur de la leptine (LEPR), le syndrome de Prader-Willi (PWS), le syndrome de Bardet-Biedl (BBS) et l'obésité hétérozygote à fort impact.

10. Procédé de l'une quelconque des revendications 1 à 9, dans lequel les molécules d'ADN double brin cibles sont extraites d'une prise de sang prélevée chez un humain.

11. Procédé de la revendication 1, comprenant :
l'aliquotage de la pluralité d'échantillons biologiques dans une pluralité de sous-groupes, de sorte qu'il n'y ait pas deux échantillons biologiques aliquotés dans la même combinaison de sous-groupes ;
la génération de données de séquençage duplex à partir de données de séquençage brutes, lesdites données de séquençage brutes étant générées à partir de la pluralité d'échantillons biologiques répartis en sous-groupes comprenant les molécules d'ADN double brin cibles, et lesdites molécules d'ADN double brin cibles comprenant un ou plusieurs variants génétiques ; et
l'identification d'une source de donneur de la ou des variants génétiques présents dans les échantillons biologiques répartis en sous-groupes en identifiant la combinaison unique de sous-groupes contenant la ou les variants génétiques.

12. Procédé de la revendication 11, dans lequel pour chaque sous-groupe, le procédé comprend en outre :
(a) la préparation d'une banque de séquençage à partir des échantillons biologiques aliquotés, ladite préparation de la banque de séquençage comprenant la ligature de molécules d'adaptateur asymétriques à la pluralité de molécules d'ADN double brin cibles dans le sous-groupe afin de générer une pluralité de molécules ADN-adaptateur ;
(b) le séquençage des premier et second brins des molécules ADN-adaptateur pour fournir une lecture de séquence de premier brin et une lecture de séquence de second brin pour chaque molécule ADN-adaptateur de la pluralité de molécules ADN-adaptateur ; et
(c) pour chaque molécule ADN-adaptateur, la comparaison de la lecture de séquence de premier brin et de la lecture de séquence de second brin afin d'identifier une ou plusieurs correspondances entre les lectures de séquences de premier et second brins afin de fournir les lectures de séquences avec correction d'erreurs pour chacune d'une pluralité de molécules d'ADN double brin cibles dans les sous-groupes, éventuellement dans lequel avant le séquençage à l'étape (b), le procédé comprend en outre la combinaison des molécules ADN-adaptateur à partir des sous-groupes ; et éventuellement dans lequel
les molécules d'adaptateur présentent une séquence d'indexation ;
chaque sous-groupe est étiqueté à l'aide d'une séquence d'indexation unique ; et
dans lequel l'identification de la combinaison unique de sous-groupes comprend l'identification de la séquence d'indexation associée à chaque variant génétique du ou des variants génétiques, et comprenant en outre éventuellement le référencement croisé de la séquence d'indexation associée à chaque variant génétique avec la combinaison de sous-groupes dans laquelle chaque échantillon biologique est aliquoté pour identifier la source de donneur.

13. Procédé de l'une quelconque des revendications 11 à 12, dans lequel la génération de la séquence avec correction d'erreurs lue pour chacune d'une pluralité de molécules d'ADN double brin cibles dans les sous-groupes comprend en outre l'enrichissement sélectif d'un ou de plusieurs loci génomiques ciblés avant le séquençage pour fournir une pluralité de molécules ADN-adaptateur enrichies.

14. Procédé de l'une quelconque des revendications 1 à 13, dans lequel le nombre de sous-groupes est ou comprend (a) 10, 11, 12, 13, 14, 15, 16, 17, 18, 19, 20, 21, 22, 23, 24, 25, 26, 27, 28, 29, 30, 31, 32, 33,, 35, 36, 37, 38, 39, 40, 42, 45, 47, 50, 52, 55, 57, 60, 62, 65, 67, ou 70 sous-groupes ou (b) entre environ 15 et environ 40 sous-groupes, entre environ 30 et environ 50 sous-groupes, entre environ 35 et environ 55 sous-groupes, entre environ 40 et environ 60 sous-groupes, ou plus de 60 sous-groupes.
